# EUROPÄISCHE PATENTSCHRIFT

(11) **EP 3 580 201 B1**
(45) Veröffentlichungstag und Bekanntmachung des Hinweises auf die Patenterteilung: **30.12.2020**
(21) Anmeldenummer: 18701429.5
(22) Anmeldetag: 18.01.2018
(51) Int. Cl.: C07C 311/21, B41M 5/155, B41M 5/333, C07C 311/47, B41M 5/327

(54) **WÄRMEEMPFINDLICHES AUFZEICHNUNGSMATERIAL**
HEAT-SENSITIVE RECORDING MATERIAL
MATÉRIAU D'IMPRESSION THERMOSENSIBLE

(30) Priorität: 10.02.2017 DE 102017102702
(43) Veröffentlichungstag der Anmeldung: 18.12.2019
(73) Patentinhaber: Papierfabrik August Koehler SE, 77704 Oberkirch (DE)
(72) Erfinder: HORN, Michael, 77654 Offenburg (DE); STALLING, Timo, 77767 Appenweier (DE); ZIERINGER, Kerstin, 77855 Achern (DE)
(74) Vertreter: Held, Stephan
(86) Internationale Anmeldenummer: PCT/EP2018/051208
(87) Internationale Veröffentlichungsnummer: WO 2018/145874

(56) Entgegenhaltungen:
- EP-A1- 0 071 839
- EP-A1- 2 923 851
- JP-A- H11 268 422

## Beschreibung

Die Erfindung betrifft einen Farbentwickler, ein wärmeempfindliches Aufzeichnungsmaterial, umfassend ein Trägersubstrat sowie eine mindestens einen Farbbildner und mindestens diesen Farbentwickler enthaltende wärmeempfindliche farbbildende Schicht, sowie die Verwendung des im wärmeempfindlichen Aufzeichnungsmaterial enthaltenen phenolfreien Farbentwicklers.

Wärmeempfindliche Aufzeichnungsmaterialien für die Thermodirekt-Druckanwendung, die eine auf einem Trägersubstrat aufgebrachte wärmeempfindliche farbbildende Schicht (Thermoreaktionsschicht) aufweisen, sind seit langem bekannt. In der wärmeempfindlichen farbbildenden Schicht liegen üblicherweise ein Farbbildner und ein Farbentwickler vor, die unter Wärmeeinwirkung miteinander reagieren und so zu einer Farbentwicklung führen. Weit verbreitet sind preisgünstige phenolische Farbentwickler, wie beispielsweise Bisphenol A und Bisphenol S, mit welchen wärmeempfindliche Aufzeichnungsmaterialien erhalten werden können, die für zahlreiche Anwendungen ein akzeptables Leistungsprofil aufweisen. Ebenfalls bekannt sind wärmeempfindliche Aufzeichnungsmaterialien, die in der wärmeempfindlichen farbbildenden Schicht einen nicht-phenolischen Farbentwickler enthalten. Diese wurden entwickelt, um die Beständigkeit des Schriftbildes zu verbessern, insbesondere auch dann, wenn das bedruckte wärmeempfindliche Aufzeichnungsmaterial über längere Zeit gelagert wird oder mit hydrophoben Stoffen, wie weichmacherhaltigen Materialien oder Ölen, in Kontakt kommt. Insbesondere vor dem Hintergrund der öffentlichen Diskussionen über das toxische Potenzial bisphenolischer Chemikalien ist das Interesse an nicht-phenolischen Farbentwicklern stark angestiegen. Hierbei war es Ziel, die Nachteile der bisphenolischen Farbentwickler zu vermeiden, allerdings sollten die technischen Leistungseigenschaften, die mit phenolischen Farbentwicklern erzielt werden können, zumindest beibehalten, vorzugsweise aber gesteigert werden.

Der umfangreiche Stand der Technik zu nicht-phenolischen Farbentwicklern lässt trotz der großen chemischen Diversität dieser Stoffe gemeinsame strukturelle Merkmale erkennen.

So ist eine 1,3-disubstituierte (Thio)Ureido-Substruktur (Y-NH-C(X)-NH-Z mit X = S, O) ein gemeinsames Merkmal zahlreicher nicht-phenolischer Farbentwickler. Durch passende Wahl der Gruppen Y und Z können die für die Eignung als Farbentwickler relevanten funktionellen Eigenschaften moduliert werden.

Weit verbreitet sind Farbentwickler mit Sulfonyl-Harnstoff-Strukturen (-SO₂-NH-CO-NH-), da diese relativ leicht herstellbar sind und die mit ihnen hergestellten wärmeempfindlichen Aufzeichnungsmaterialien relativ gute anwendungstechnische Eigenschaften aufweisen.

Die EP 0 526 072 A1 und die EP 0 620 122 B1 offenbaren Farbentwickler aus der Klasse der aromatischen Sulfonyl-(Thio)Harnstoffe. Mit diesen können wärmeempfindliche Aufzeichnungsmaterialien erhalten werden, die sich durch eine relativ hohe Bildbeständigkeit auszeichnen. Ferner weisen die auf diesen Farbentwicklern basierenden wärmeempfindlichen Aufzeichnungsmaterialien eine brauchbare thermische Empfindlichkeit bei guter Oberflächenweiße auf, so dass es bei entsprechender Gestaltung der Rezeptur der wärmeempfindlichen farbbildenden Schicht vergleichsweise leicht möglich ist, hohe Druckdichten unter Verwendung handelsüblicher Thermodrucker zu erzeugen.

Die WO 0 035 679 A1 offenbart aromatische und heteroaromatische Sulfonyl(thio) harnstoffverbindungen (X = S oder O) und/oder Sulfonyl-Guanidine (X = NH) der Formel

Ar'-SO₂-NH-C(X)-NH-Ar,

wobei Ar durch eine zweiwertige Linkergruppe an weitere aromatische Gruppen geknüpft ist. Ein in der Praxis weit verbreiteter nicht-phenolischer Entwickler aus dieser Klasse, 4-Methyl-*N*-(((3-(((4-methylphenyl)sulfonyl)oxy)phenyl)amino)carbonyl)benzolsulfonamid (Handelsname Pergafast 201®, BASF), zeichnet sich durch Ausgewogenheit der anwendungstechnischen Eigenschaften der mit diesem Farbentwickler hergestellten wärmeempfindlichen Aufzeichnungsmaterialien aus. Insbesondere besitzen diese eine gute dynamische Ansprechempfindlichkeit und eine im Vergleich zu mit (bis)phenolischen Farbentwicklern erhaltenen Aufzeichnungsmaterialien hohe Beständigkeit des Ausdrucks gegenüber hydrophoben Stoffen. Jede Neuentwicklung muss sich mit dem Leistungsspektrum dieses etablierten nicht-phenolischen Farbentwicklers messen lassen.

Sulfonylharnstoffe neigen in Gegenwart von Wasser bzw. Feuchtigkeit und in der Wärme zu hydrolytischen Zersetzungsreaktionen. Dies führt dazu, dass wärmeempfindliche Aufzeichnungsmaterialien bei Lagerung in unbedrucktem Zustand unter Bedingungen erhöhter Luftfeuchtigkeit und/oder Temperatur eine teilweise Zersetzung des Farbentwicklers erfahren können.

Da die Schreibleistung (dynamische Ansprechempfindlichkeit) wärmeempfindlicher Aufzeichnungsmaterialien auch von der Menge des in der wärmeempfindlichen Schicht vorliegenden Farbentwicklers abhängt, verliert ein über längere Zeiträume derart gelagertes wärmeempfindliches Aufzeichnungsmaterial einen Teil des Farbentwicklers und dadurch seine Schreibleistung.

Die oben angesprochene Möglichkeit der Modulation der Eigenschaften der 1,3'-disubstituierten Harnstoffeinheit kann auch durch Einbeziehung nicht unmittelbar an die Ureido-Einheit gebundener, aber mit dieser über eine Linkergruppe, sei es in konjugativer Verbundenheit stehender oder in synergetisch vorteilhafter Stellung befindlicher Gruppierungen erreicht werden.

Dieser Ansatz wurde beispielsweise in der JP H 11 268 421 verfolgt. Diese offenbart die Kombination einer (Thio)Ureido-Einheit mit einer Sulfamoyl-Gruppe (-NH-SO₂-) der Formel

Ar¹-NH-SO₂-Ar-NH-C(X)-NH-Ar²,

wobei Ar, Ar¹ und Ar² einkernige aromatische Gruppen darstellen und X = S oder O ist.

Die JP 11 268 422 A offenbart Strukturen der Formel

Ar¹-NH-SO₂-Ar-NH-C(O)-NH-A-NH-C(O)-NH-Ar-SO₂-NH-Ar¹,

wobei A aromatische oder aliphatische Gruppen sein können. Auch Strukturen mit primären -SO₂-NH₂-Gruppen werden in diesem Kontext beschrieben (EP 0 693 386 A1).

Die EP 2 923 851 A1 offenbart Farbentwicklerstrukturen der Formel

Ar¹-SO₂-NH-Ar-NH-C(O)-NH-Ar².

Obwohl wärmeempfindliche Aufzeichnungsmaterialien auf Basis dieser Farbentwickler eine gute dynamische Sensitivität gewährleisten, ist die Stabilität des Farbkomplexes, insbesondere gegenüber Weichmachern oder Klebstoffen, verbesserungsbedürftig.

Schon frühzeitig wurde versucht, die Leistungsfähigkeit nicht-phenolischer Farbentwickler durch Verwendung von Farbentwicklerstrukturen, die mehr als nur eine der für den Farbbildungsprozess relevanten Struktureinheiten enthalten, zu verbessern.

Die JP H 06 227 142 A offenbart Farbentwickler mit zwei, drei oder mehr (Thio)Harnstoffeinheiten (Bis-, Tris-, Polykis-Harnstoffe) in der Verbindung (Ar¹-NH-C(X)-NH)ₙ-A, die an eine meist aromatische Einheit A gebunden sind (X = S oder O).

Strukturell ähnlich aufgebaut sind die in der EP 633 145 A1, der JP H 082 109 A, der JP H 08 244 355 A, der JP H 8 244 355 A und der JP H 11 268 422 A beschriebenen Farbentwickler.

Dieser Ansatz wurde durch die Verwendung von -OH, -CO₂H, -SO₂NH₂ und -SO₂NH-Aryl-Gruppen substituierter Ar¹-Reste vielfach variiert (JP H 082 109 A, JP H 08 244 355 A, JP 08 197 851 A, JP H 10 230 681 A, JP H 11 268 422 A). Obwohl bis- oder polykis-Harnstoffderivate gute H-Brücken-Akzeptor- und Donator-Eigenschaften besitzen und daher geeignet sind, den Farbkomplex zu stabilisieren, bedingen die sich zwischen den einzelnen Harnstoffeinheiten ausbildenden Wasserstoffbrücken-Netzwerke einen relativ hohen Schmelzpunkt und eine geringe Löslichkeit dieser Stoffe in typischen thermischen Lösemitteln aus der wärmeempfindlichen Schicht mit der Folge, dass die thermische Ansprechempfindlichkeit (sog. dynamische Sensitivität) der mit diesen Farbentwicklern hergestellten wärmeempfindlichen Aufzeichnungsmaterialien zu wünschen übrig lässt.

Aufgabe der vorliegenden Erfindung ist es daher, vorstehend geschilderte Nachteile des Standes der Technik zu beheben. Insbesondere besteht die Aufgabe der vorliegenden Erfindung darin, einen Farbentwickler und ein diesen enthaltendes wärmeempfindliches Aufzeichnungsmaterial bereitzustellen, welches ein ausgewogenes anwendungstechnisches Eigenschaftsprofil hat und eine praxistaugliche Druckdichte erreicht, vergleichbar jener, die mit etablierten nicht-phenolischen Farbentwicklern des Standes der Technik möglich sind, dabei aber eine hohe Beständigkeit des Druckbildes insbesondere gegenüber hydrophoben Agenzien sicherstellt, ohne auf spezielle Rezepturbestandteile in der wärmeempfindlichen Funktionsschicht wie Alterungsschutzmittel oder spezielle Schmelzhilfsmittel mit eingeschränkter Verfügbarkeit und hohem Preis angewiesen zu sein. Auch ist es Aufgabe der vorliegenden Erfindung, ein wärmeempfindliches Aufzeichnungsmaterial zur Verfügung zu stellen, welches in der Lage ist, die anwendungstechnisch notwendigen funktionellen Eigenschaften (insbesondere die thermische Ansprechempfindlichkeit) auch unter Bedingungen der Lagerung über lange Zeiträume unter extremen Klimabedingungen des unbedruckten Materials zu gewährleisten.

Erfindungsgemäß wird diese Aufgabe durch den Einsatz einer Verbindung nach Anspruch 1 in einem wärmeempfindlichen Aufzeichnungsmaterial nach Anspruch 13 gelöst.

Die Verbindung nach Anspruch 1 besitzt die Formel (I),

Ar(NHSO₂Ar¹)ₗ(SO₂NHAr¹)ₘ(NHC(O)NHAr²)ₙ (I),

wobei
l und m unabhängig voneinander 0, 1, 2, 3 und/oder 4 sind und die Summe l+m gleich oder größer als 1 ist,
n 2, 3, 4 oder 5 ist,
Ar ein (l+m+n)-fach substituierter Benzol-Rest ist,
Ar¹ ein unsubstituierter oder substituierter aromatischer Rest ist und
Ar² ein unsubstituierter oder substituierter Phenyl-Rest oder ein Benzoyl-Rest ist.

Vorzugsweise ist die Summe l+m 1 oder 2 und ganz besonders bevorzugt 1, da derartige Verbindungen synthetisch leichter zugänglich sind.

Vorzugsweise ist l oder m 0, da derartige Verbindungen synthetisch leichter zugänglich sind.

Vorzugsweise ist l 0 oder 1, besonders bevorzugt 1, da derartige Verbindungen synthetisch leichter zugänglich sind.

Vorzugsweise ist m 0, 1 oder 2, vorzugsweise 0 oder 1 und besonders bevorzugt 0, da derartige Verbindungen synthetisch leichter zugänglich sind.

Vorzugsweise ist n 2, da derartige Verbindungen synthetisch leichter zugänglich sind.

Besonders bevorzugt ist eine Verbindung der Formel (I), wobei l 1, m 0 und n 2 ist.

Ebenfalls besonders bevorzugt ist eine Verbindung der Formel (I), wobei l 0, m 1 und n 2 ist.

Auch eine Verbindung der Formel (I), wobei l 0, m 2 und n 2 ist, ist besonders bevorzugt.

Vorzugsweise ist Ar ein 3- oder 4-fach substituierter Benzol-Rest.

Wie vorstehend erwähnt, ist Ar¹ ein unsubstituierter oder substituierter aromatischer Rest. Die Substitution kann einfach oder mehrfach mit gleichen oder unterschiedlichen Resten erfolgen. Der unsubstituierte oder substituierte aromatische Rest ist vorzugsweise ein Phenyl-Rest oder ein 4-Alkoxycarbonylphenyl-Rest. In einer besonders bevorzugten Ausführungsform handelt es sich bei dem Phenyl-Rest um einen einfach substituierten Phenyl-Rest. Derartige Verbindungen haben den Vorteil, dass diese synthetisch leichter zugänglich sind.

Der einfach substituierte Phenyl-Rest ist vorzugsweise mit einem C₁-C₅-Alkyl-, einem Alkenyl-, einem Alkinyl-, einem Benzyl-, einem RO-, einem Halogen-, Formyl-, einem ROC-, einem RO₂C-, einem CN-, einem NO₂-, einem R-SO₂O-, einem RO-SO₂-, einem R-NH-SO₂-, einem R-SO₂-NH-, einem R-NH-CO-NH-, einem R-SO₂-NH-CO-NH-, einem R-NH-CO-NH-R- oder einem R-CO-NH-Rest substituiert, wobei R ein C₁-C₅-Alkyl-, ein Alkenyl-, ein Alkinyl-, ein Phenyl-, ein Tolyl- oder ein Benzyl-Rest ist.

Ganz besonders bevorzugt ist der einfach substituierte Phenyl-Rest mit einem 4-C₁-C₅-Alkyl-, vorzugsweise einem 4-Methyl-, 4-Ethyl-, 4-n-Propyl- oder 4-iso-Propyl-Rest, einem 4-RO- oder einem 4-(RO₂C)-Rest, wobei R ein C₁ bis C₅-Alkyl-Rest, vorzugsweise ein Methyl- oder ein Ethyl-Rest ist, substituiert. Auch kann der Phenyl-Rest vorzugsweise mit einem Halogen-Rest, besonders bevorzugt einem Chlorid-Rest, substituiert sein.

Besonders bevorzugt ist Ar¹ ein Phenyl- oder ein 4-Tolyl-Rest, da derartige Verbindungen synthetisch leichter zugänglich sind.

Wie vorstehend erwähnt, handelt es sich bei Ar² um einen unsubstituierten oder substituierten Phenyl-Rest oder einen Benzoyl-Rest. Diese sind vorzugsweise mit einem C₁ bis C₄-Alkyl-, einem Halogen-, einem CX₃-, einem Formyl-, einem ROC-, einem RO₂C-, einem CN-, einem NO₂- oder einem RO-Rest substituiert, wobei X ein Halogen-Rest und R ein C₁ bis C₅-Alkyl-, vorzugsweise ein Methyl-Rest, ein Phenyl- oder ein Tolyl-Rest ist.

Besonders bevorzugt ist Ar² ein Phenyl-, ein 4-Tolyl- oder ein 4-Acetylphenyl-Rest. Dies hat den Vorteil, dass die anwendungstechnischen Leistungen, insbesondere die Weichmacherbeständigkeit, sehr gut sind.

Besonders bevorzugte einzelne Verbindungen der Formel (I) sind in folgender Tabelle 1 dargestellt.

**Tabelle 1: Bevorzugte Verbindungen der Formel (I) mit den angegebenen Bedeutungen für die Reste Ar, Ar¹ und Ar² sowie für die Indizes l, m und n**

| Ar | Ar¹ | Ar² | l | m | n |
|---|---|---|---|---|---|
| Benzol-triyl- | Phenyl- | Phenyl- | 1 | 0 | 2 |
| Benzol-triyl- | Phenyl- | Phenyl- | 0 | 1 | 2 |
| Benzol-triyl- | einfach substituierter Phenyl- | Phenyl- | 1 | 0 | 2 |
| Benzol-triyl- | C₁-C₅-Alkyl substituierter Phenyl | Phenyl- | 1 | 0 | 2 |
| Benzol-triyl- | einfach substituierter Phenyl- | RO-substituierter Phenyl- | 1 | 0 | 2 |
| Benzol-triyl- | C₁-C₅-Alkyl substituierter Phenyl- | RO-substituierter Phenyl- | 1 | 0 | 2 |
| Benzol-triyl- | C₁-C₅-Alkyl substituierter Phenyl- | C₁-C₄-Alkyl substituierter Phenyl- | 1 | 0 | 2 |
| Benzol-triyl- | C₁-C₅-Alkyl substituierter Phenyl- | PhOsubstituierter Phenyl- | 1 | 0 | 2 |
| Benzol-triyl- | C₁-C₅-Alkyl substituierter Phenyl- | Halogensubstituierter Phenyl- | 1 | 0 | 2 |
| Benzol-triyl- | C₁-C₅-Alkyl substituierter Phenyl- | CX₃-su bstitu ierter Phenyl- | 1 | 0 | 2 |
| Benzol-triyl- | Halogen-substituierter Phenyl- | Phenyl- | 1 | 0 | 2 |
| Benzol-triyl- | RO-substituierter Phenyl- | Phenyl- | 1 | 0 | 2 |
| Benzol-triyl- | C₁-C₅-Alkyl substituierter Phenyl- | ROCsubstituierter Phenyl | 1 | 0 | 2 |
| Benzol-triyl- | C₁-C₅-Alkyl substituierter Phenyl- | Benzoyl- | 1 | 0 | 2 |
| Benzol-triyl- | RO₂C-substituierter Phenyl- | Phenyl- | 0 | 1 | 2 |
| Benzol-tetryl- | Phenyl- | Phenyl- | 0 | 2 | 2 |

Unter einem Benzol-triyl-Rest wird ein dreifach-substituierter Benzol-Rest, unter einem Benzol-tetryl-Rest ein vierfach-substituierter Benzol-Rest verstanden. Die Dreifach-Substitution erfolgt vorzugsweise in 1,2,3-, 1,2,4-, 1,2,5-, 1,2,6- oder 1,3,4-Stellung. Die Vierfach-Substitution erfolgt vorzugsweise in 1,3,4,6-Stellung.

In obiger Tabelle 1 bedeuten R vorzugsweise einen C₁-C₄-Alkyl- und X einen Halogen-Rest, besonders bevorzugt einen Fluorid-Rest.

Die Herstellung der erfindungsgemäßen Verbindung der Formel (I) kann nach an sich bekannten Methoden erfolgen.

Nachfolgendes Reaktionsschema 1 veranschaulicht einen möglichen Syntheseweg für die erfindungsgemäße Verbindung der Formel (I) am Beispiel der Verbindungen **I** bis **XVIII** (siehe Tabelle 2).

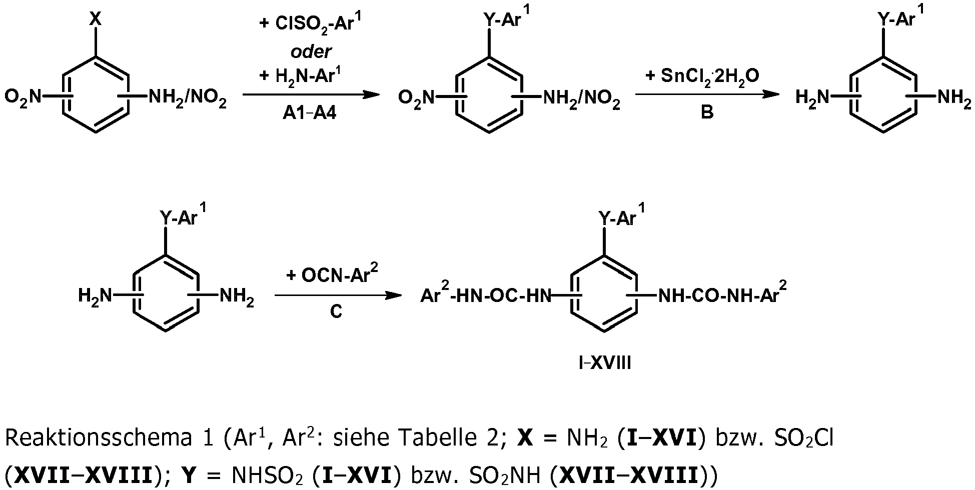

Die unter die erfindungsgemäße Verbindung der Formel (I) fallenden Verbindungen **I** und **II** (siehe Tabelle 2) können ausgehend von 2,6-Dinitroanilin, das zunächst gemäß nachfolgendem Reaktionsschema 2 in 1,2-Diamino-3-nitrobenzol (V. Milata, J. Saloň, Org. Prep. Proceed. Int., 31 (3), 347 (1999)) und dann nach den beschriebenen Methoden in das Endprodukt überführt wird, hergestellt werden.

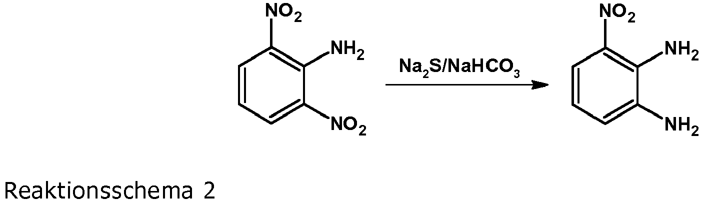

Die unter die erfindungsgemäße Verbindung der Formel (I) fallende Verbindung **XIX** (siehe Tabelle 2) kann ausgehend von 1,3-Phenylendiamin-Dihydrochlorid, das zunächst gemäß nachfolgendem Reaktionsschema 3 in das entsprechende Bis-Aminosulfonylchlorid (G. Barnikow, K. Krüger, G. Hilgetag, Z. Chem., 6 (7), 262 (1966)) und dann nach den beschriebenen Methoden in das Endprodukt überführt wird, hergestellt werden.

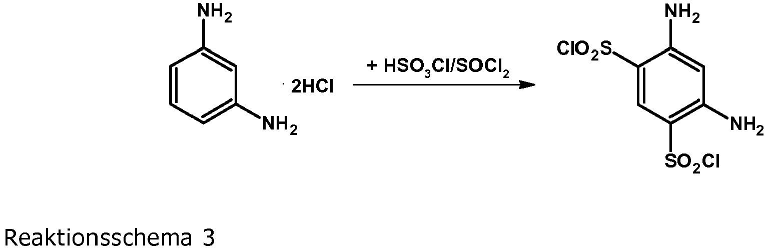

Wie vorstehend bereits erwähnt, betrifft die vorliegende Erfindung auch ein wärmeempfindliches Aufzeichnungsmaterial, umfassend ein Trägersubstrat, mindestens einen Farbbildner und mindestens einen phenolfreien Farbentwickler enthaltende wärmeempfindliche farbbildende Schicht, wobei der mindestens eine phenolfreie Farbentwickler die Verbindung der vorstehend beschriebenen Formel (I) ist.

Die Verbindung der Formel (I) liegt vorzugsweise in einer Menge von etwa 3 bis etwa 35 Gew.-%, besonders bevorzugt in einer Menge von etwa 10 bis etwa 25 Gew.-% bezogen auf den gesamten Feststoffgehalt der wärmeempfindlichen Schicht, vor.

Die Auswahl des Trägersubstrates ist nicht kritisch. Allerdings ist es bevorzugt, als Trägersubstrat Papier, synthetisches Papier und/oder eine Kunststoff-Folie einzusetzen.

Gegebenenfalls liegt zwischen dem Trägersubstrat und der wärmeempfindlichen Schicht mindestens eine weitere Zwischenschicht vor, wobei dieser die Aufgabe zukommt, die Oberflächenglätte des Trägers für die wärmeempfindlichen Schicht zu verbessern und eine Wärmebarriere zwischen dem Trägersubstrat und der wärmeempfindlichen Schicht zu gewährleisten.

Vorzugsweise kommen in dieser Zwischenschicht organische Hohlkugelpigmente und/oder kalzinierte Kaoline zum Einsatz.

Auch kann mindestens eine oberhalb der wärmeempfindlichen Schicht angeordnete Schutzschicht und/oder mindestens eine die Bedruckbarkeit begünstigende Schicht im erfindungsgemäßen wärmeempfindlichen Aufzeichnungsmaterial vorliegen, wobei diese Schichten auf der Vorder- oder Rückseite des Substrats aufgebracht werden können.

Hinsichtlich der Wahl des Farbbildners unterliegt die vorliegende Erfindung ebenfalls keinen wesentlichen Einschränkungen. Bevorzugt ist der Farbbildner jedoch ein Farbstoff vom Triphenylmethan-Typ, vom Fluoran-Typ, vom Azaphthalid-Typ und/oder vom Fluoren-Typ. Ein ganz besonders bevorzugter Farbbildner ist ein Farbstoff vom Fluoran-Typ, da dieser dank der Verfügbarkeit und der ausgewogenen anwendungsbezogenen Eigenschaften die Bereitstellung eines Aufzeichnungsmaterials mit attraktivem Preis-Leistungsverhältnis ermöglicht.

Besonders bevorzugte Farbstoffe vom Fluoran-Typ sind:
3-Diethylamino-6-methyl-7-anilinofluoran,
3-(*N*-ethyl-*N*-p-toluidinamino)-6-methyl-7-anilinofluoran,
3-(*N*-ethyl-*N*-isoamylamino)-6-methyl-7-anilinofluoran,
3-Diethylamino-6-methyl-7-(o,p-dimethylanilino)fluoran,
3-Pyrrolidino-6-methyl-7-anilinofluoran,
3-(Cyclohexyl-*N*-methylamino)-6-methyl-7-anilinofluoran,
3-Diethylamin-7-(m-trifluoromethylanilino)fluoran,
3-*N*-n-Dibutylamin-6-methyl-7-anilinofluoran,
3-Diethylamino-6-methyl-7-(m-methylanilino)fluoran,
3-*N*-n-dibutylamin-7-(o-chloroanilino) fluoran,
3-(*N*-Ethyl-*N*-tetrahydrofurfurylamin)-6-methyl-7-anilino-fluoran,
3-(*N*-methyl-*N*-propylamin)-6-methyl-7-anilinofluoran,
3-(*N*-ethyl-*N*-ethoxypropylamin)-6-methyl-7-anilinofluoran,
3-(*N*-ethyl-*N*-isobutylamin)-6-methyl-7-anilinofluoran und/oder
3-Dipentylamin-6-methyl-7-anilinofluoran.

Die Farbbildner können als Einzelstoffe als auch als beliebige Gemische zweier oder mehrerer Farbbildner zur Anwendung kommen, vorausgesetzt, die wünschenswerten anwendungstechnischen Eigenschaften der Aufzeichnungsmaterialien leiden darunter nicht.

Der Farbbildner liegt vorzugsweise in einer Menge von etwa 5 bis etwa 30, besonders bevorzugt in einer Menge von etwa 8 bis etwa 20, bezogen auf den gesamten Feststoffgehalt der wärmeempfindlichen Schicht, vor.

Zur Steuerung spezieller anwendungstechnischer Eigenschaften kann es vorteilhaft sein, wenn mindestens zwei der der allgemeinen Formel (I) entsprechenden Verbindungen als Farbentwickler in der wärmeempfindlichen Schicht vorliegen.

Desgleichen können ein oder mehrere weitere (bis)phenolische oder nicht-phenolische Farbentwickler zusätzlich zu Verbindungen der Formel (I) in der wärmeempfindlichen farbbildenden Schicht vorliegen.

Neben dem mindestens einem Farbbildner und dem mindestens einem Farbentwickler können in der wärmeempfindlichen farbbildenden Schicht ein oder mehrere Sensibilisierungsmittel, auch thermische Lösungsmittel oder Schmelzhilfsmittel genannt, vorliegen, was den Vorteil hat, dass die Steuerung der thermischen Druckempfindlichkeit leichter zu realisieren ist.

Generell kommen als Sensibilisierungsmittel vorteilhafterweise kristalline Stoffe in Betracht, deren Schmelzpunkt zwischen etwa 90 und etwa 150 °C liegt und die im geschmolzenen Zustand die farbbildenden Komponenten (Farbbildner und Farbentwickler) lösen, ohne die Ausbildung des Farbkomplexes zu stören.

Vorzugsweise ist das Sensibilisierungsmittel ein Fettsäureamid, wie Stearamid, Beheneamid oder Palmitamid, ein Ethylen-bis-fettsäureamid, wie *N,N'*-Ethylen-bis-stearinsäureamid oder *N,N'*-Ethylen-bis-ölsäureamid, ein Fettsäurealkanolamid, wie *N*-(Hydroxymethyl)stearamid, *N*-Hydroxymethylpalmitamid oder Hydroxyethylstearamid, ein Wachs, wie Polyethylenwachs oder Montanwachs, ein Carbonsäureester, wie Dimethylterephthalat, Dibenzylterephthalat, Benzyl-4-benzyloxybenzoat, Di-(4-methylbenzyl)oxalat, Di-(4-chlorbenzyl)oxalat oder Di-(4-benzyl)oxalat, Ketone, wie 4-Acetylbiphenyl, ein aromatischer Ether, wie 1,2-Diphenoxy-ethan, 1,2-Di-(3-methylphenoxy)ethan, 2-Benzyloxynaphthalin, 1,2-Bis-(phenoxymethyl)benzol oder 1,4-Diethoxynaphthalin, ein aromatisches Sulfon, wie Diphenylsulfon, und/ oder ein aromatisches Sulfonamid, wie 4-Toluolsulfonamid, Benzolsulfonanilid oder *N*-Benzyl-4-toluolsulfonamid oder aromatische Kohlenwasserstoffe, wie 4-Benzylbiphenyl.

Das Sensibilisierungsmittel liegt vorzugsweise in einer Menge von etwa 10 bis etwa 40, besonders bevorzugt in einer Menge von etwa 15 bis etwa 25, bezogen auf den gesamten Feststoffgehalt der wärmeempfindlichen Schicht, vor.

In einer weiteren bevorzugten Ausführungsform liegt neben dem Farbbildner, dem phenolfreien Farbentwickler und dem Sensibilisierungsmittel optional mindestens ein Stabilisator (Alterungsschutzmittel) in der wärmeempfindlichen farbbildenden Schicht vor.

Bei dem Stabilisator handelt es sich vorzugsweise um sterisch gehinderte Phenole, besonders bevorzugt um 1,1,3-Tris-(2-methyl-4-hydroxy-5-cyclohexyl-phenyl)butan, 1,1,3-Tris-(2-methyl-4-hydroxy-5-tert-butylphenyl)butan, 1,1-Bis-(2-methyl-4-hydroxy-5-tert-butyl-phenyl)butan.

Auch Harnstoff-Urethan-Verbindungen (Handelsprodukt UU) oder vom 4,4'-Dihydroxydiphenylsulfon abgeleitete Ether, wie 4-Benzyloxy-4'-(2-methylglycidyloxy)-diphenylsulfon (Handelsname NTZ-95®, Nippon Soda Co. Ltd.), oder oligomere Ether (Handelsname D90®, Nippon Soda Co. Ltd.) sind als Stabilisatoren im erfindungsgemäßen Aufzeichnungsmaterial einsetzbar.

Der Stabilisator liegt vorzugsweise in einer Menge von 0,2 bis 0,5 Gew.-Teilen, bezogen auf den mindestens einen phenolfreien Farbentwickler der Verbindung der Formel (I), vor.

In einer weiteren bevorzugten Ausführungsform liegt in der wärmeempfindlichen farbbildenden Schicht mindestens ein Bindemittel vor. Bei diesem handelt es sich vorzugsweise um wasserlösliche Stärken, Stärkederivate, stärkebasierte Biolatices vom EcoSphere®-Typ, Methylcellulose, Hydroxyethylcellulose, Carboxymethylcellulosen, partiell oder vollständig verseifte Polyvinylalkohole, chemisch modifizierte Polyvinylalkohole oder Styrolmaleinsäureanhydrid-Copolymere, Styrolbutadien-Copolymere, Acrylamid-(Meth)acrylat-Copolymere, Acrylamid-Acrylat-Methacrylat-Terpolymere, Polyacrylate, Poly(meth)-acrylsäureester, Acrylat-Butadien-Copolymere, Polyvinylacetate und/oder Acrylnitril-Butadien-Copolymere.

In einer weiteren bevorzugten Ausführungsform liegt mindestens ein Trennmittel (Antihaftmittel) oder Gleitmittel in der wärmeempfindlichen farbbildenden Schicht vor. Bei diesen Mitteln handelt es sich vorzugsweise um Fettsäure-Metallsalze, wie z. B. Zinkstearat oder Calciumstearat, oder auch Behenatsalze, synthetische Wachse, z. B. in Form von Fettsäureamiden, wie z. B. Stearinsäureamid und Behensäureamid, Fettsäurealkanolamide, wie z. B. Stearinsäure-methylolamid, Paraffinwachse verschiedener Schmelzpunkte, Esterwachse unterschiedlicher Molekulargewichte, Ethylenwachse, Propylenwachse unterschiedlicher Härten und/oder natürliche Wachse, wie z. B. Carnaubawachs oder Montanwachs.

Das Trennmittel liegt vorzugsweise in einer Menge von etwa 1 bis etwa 10, besonders bevorzugt in einer Menge von etwa 3 bis etwa 6, bezogen auf den gesamten Feststoffgehalt der wärmeempfindlichen Schicht, vor.

In einer weiteren bevorzugten Ausführungsform enthält die wärmeempfindliche farbbildende Schicht Pigmente. Der Einsatz dieser hat unter anderem den Vorteil, dass diese auf ihrer Oberfläche die im thermischen Druckprozess entstehende Chemikalien-Schmelze fixieren können. Auch kann über Pigmente die Oberflächenweiße und Opazität der wärmeempfindlichen farbbildenden Schicht und deren Bedruckbarkeit mit konventionellen Druckfarben gesteuert werden. Schließlich besitzen Pigmente eine "Extenderfunktion", beispielsweise für die relativ teuren farbgebenden Funktionschemikalien.

Besonders geeignete Pigmente sind anorganische Pigmente, sowohl synthetischer als auch natürlicher Herkunft, vorzugsweise Clays, gefällte oder natürliche Calciumcarbonate, Aluminiumoxide, Aluminiumhydroxide, Kieselsäuren, gefällte und pyrogene Kieselsäuren (z. B. Aerodisp®-Typen), Diathomeenerden, Magnesiumcarbonate, Talk, aber auch organische Pigmente, wie Hohlpigmente mit einer Styrol/Acrylat-Copolymer-Wand oder Harnstoff/Formaldehyd-Kondensationspolymere. Diese können alleine oder in beliebigen Mischungen verwendet werden.

Die Pigmente liegen vorzugsweise in einer Menge von etwa 20 bis etwa 50, besonders bevorzugt in einer Menge von etwa 30 bis etwa 40, bezogen auf den gesamten Feststoffgehalt der wärmeempfindlichen Schicht, vor.

Zum Steuern der Oberflächenweiße des erfindungsgemäßen wärmeempfindlichen Aufzeichnungsmaterials können optische Aufheller in die wärmeempfindliche farbbildende Schicht eingebaut werden. Bei diesen handelt es sich vorzugsweise um Stilbene.

Um bestimmte streichtechnische Eigenschaften zu verbessern, ist es im Einzelfall bevorzugt, zu den zwingenden Bestandteilen des erfindungsgemäßen wärmeempfindlichen Aufzeichnungsmaterials weitere Bestandteile, insbesondere Rheologie-Hilfsmittel, wie z. B. Verdicker und/oder Tenside, hinzuzufügen.

Das Flächenauftragsgewicht der (trockenen) wärmeempfindlichen Schicht beträgt vorzugsweise etwa 1 bis etwa 10 g/m², bevorzugt etwa 3 bis etwa 6 g/m².

In einer besonders bevorzugten Ausführungsform handelt es sich bei dem wärmeempfindlichen Aufzeichnungsmaterial um ein solches nach Anspruch 13, wobei als Farbbildner ein Farbstoff vom Fluoran-Typ eingesetzt wird und zusätzlich ein Sensibilisierungsmittel, ausgewählt aus der Gruppe bestehend aus Fettsäureamiden, aromatischen Sulfonen und/oder aromatischen Ethern, vorliegt. In dieser bevorzugten Ausführungsform ist es auch vorteilhaft, dass etwa 1,5 bis etwa 4 Gew.-Teile des phenolfreien Farbentwicklers nach Anspruch 1, bezogen auf den Farbbildner, vorliegen.

Die im Zusammenhang mit der Verbindung der Formel (I) aufgeführten bevorzugten Ausführungsformen gelten auch für das erfindungsgemäße wärmeempfindliche Aufzeichnungsmaterial.

Das erfindungsgemäße wärmeempfindliche Aufzeichnungsmaterial lässt sich mit bekannten Herstellungsverfahren gewinnen.

Es ist vorteilhaft, wenn die getrocknete wärmeempfindliche farbbildende Schicht einer Glätt-Maßnahme unterzogen wird. Hierbei ist es vorteilhaft, die Bekk-Glätte, gemessen nach ISO 5627 : 1995-03, auf etwa 100 bis etwa 1000 sec., vorzugsweise auf etwa 250 bis etwa 600 sec., einzustellen.

Die Oberflächenrauigkeit (PPS) nach ISO 8791-4 : 2008-05 liegt im Bereich von etwa 0,50 bis etwa 2,50 µm, vorzugsweise im Bereich von 1,00 und 2,00 µm.

Das erfindungsgemäße wärmeempfindliche Aufzeichnungsmaterial ist phenolfrei und für POS (point-of-sale), Etiketten- und/oder Ticket-Anwendungen gut geeignet. Es eignet sich auch zur Herstellung von Parkscheinen, Fahrkarten, Eintrittskarten, Lotto- und Wettscheinen etc., welche in Thermodirektverfahren bedruckt werden können und eine hohe Beständigkeit der darauf aufgezeichneten Bilder unter längerfristiger Lagerung, selbst unter verschärften Klimabedingungen hinsichtlich Temperatur und Umgebungsfeuchte, und beim Inkontaktbringen des Schriftbildes mit hydrophoben Stoffen, wie Weichmachern, Klebstoffen, fettigen oder öligen Stoffen, etc., benötigen.

Die mit den erfindungsmäßigen Entwicklern der Formel (I) erhaltenen wärmeempfindlichen Aufzeichnungsmaterialien büßen ihre Fähigkeit, hohe Bilddichten zu erzeugen, auch nach wochenlanger Lagerung der unbedruckten Materialien bei hoher Umgebungsluftfeuchte und Temperatur, kaum ein (gute Lagerfähigkeit).

Zusammenfassend kann festgehalten werden, dass es sich überraschenderweise gezeigt hat, dass es möglich ist, mit den erfindungsgemäßen Farbentwicklern der Formel (I) wärmeempfindliche Aufzeichnungsmaterialien zu erhalten, welche sich durch herausragende Beständigkeit des Schriftbildes gegenüber hydrophoben Agenzien auszeichnen und mit denen eine gute Qualität des Druckbildes (hohe o.D. des Druckbildes) zu erreichen ist. Des Weiteren ist die Langzeitlagerfähigkeit der erfindungsgemäßen wärmeempfindlichen Aufzeichnungsmaterialien hervorragend. Selbst bei Lagerung in unbedrucktem Zustand über mehrere Wochen bei hohen Umgebungsfeuchten oder Temperaturen leidet die erzielte optische Dichte beim Bedrucken im Thermodrucker kaum.

Ohne an diese Theorie gebunden zu sein, scheint insbesondere die molekulare enge nachbarschaftliche Anordnung einer erhöhten Zahl (≥3) für den Farbbildungs- und Farbstabilisierungsprozess essentieller funktioneller Gruppen (hohe molekulare Dichte der maßgeblichen funktionellen Gruppen) unmittelbar an ein und derselben aromatischen Einheit entsprechend der allgemeinen Formel (I) eine wichtige Rolle zu spielen.

Die Erfindung wird nachfolgend anhand nicht beschränkter Beispiele im Detail erläutert.

Als Vergleichsentwickler wurden nicht-phenolische Farbentwickler des Standes der Technik herangezogen: Zwei isomere Harnstoffderivate mit *N*-Phenyl-2-(3-phenylureido)benzolsulfonamid (**Y**) und *N*-(2-(3-Phenylureido)phenyl)benzolsulfonamid (**Z**) sowie ein Sulfonylharnstoff mit Pergafast 201®, BASF (PF201).

### Beispiele:

Herstellung der erfindungsgemäßen Verbindungen der Formel (I).

Die Verbindungen **I** bis **XIX** (Tabelle 2) wurden wie folgt hergestellt:

### Stufe A1 - Herstellung der Sulfonamide

Zu einer Lösung aus 20 mmol aromatischem Diamin und 20 mmol Pyridin in 125 mL Dichlormethan wird eine Lösung aus 10 mmol des entsprechenden Sulfonylchlorids in 75 mL Dichlormethan bei 0 °C unter Rühren tropfenweise zugegeben. Die Reaktionslösung wird 16 Stunden bei Raumtemperatur gerührt und anschließend mit 100 mL Wasser versetzt. Die organische Phase wird abgetrennt und mit 250 mL einer 5%igen wässrigen Natriumhydroxid-Lösung versetzt. Die wässrige Phase wird mit 100 mL Dichlormethan gewaschen und durch Zugabe 25%iger Salzsäure neutral gestellt. Nach mehrmaliger Extraktion mit 100 mL Dichlormethan werden die vereinigten organischen Phasen mit 200 mL Wasser gewaschen und über Magnesiumsulfat getrocknet. Nach Entfernen des Lösungsmittels im Vakuum bleibt das Sulfonamid als Feststoff zurück. Die Sulfonamide wurden ohne weitere Aufreinigung in der Stufe B verwendet.

### Stufe A2 - Herstellung der Sulfonamide

Zu einer Lösung aus 80 mmol aromatischem Amin und 240 mmol Kaliumcarbonat in 500 mL Dichlorethan wird eine Lösung aus 80 mmol des entsprechenden Sulfonylchlorids in 150 mL Dichlorethan bei Raumtemperatur unter Rühren tropfenweise zugegeben. Das Reaktionsgemisch wird sechs Stunden refluxiert und anschließend mit 300 mL Ethylacetat und 300 mL Wasser versetzt. Die wässrige Phase wird durch Zugabe 25%iger Salzsäure sauer gestellt. Die Phasen werden getrennt. Nach mehrmaliger Extraktion der wässrigen Phase mit 200 mL Ethylacetat werden die vereinigten organischen Phasen mit 200 mL Wasser gewaschen und über Magnesiumsulfat getrocknet. Nach Entfernen der Lösungsmittel im Vakuum bleibt das Sulfonamid als Feststoff zurück. Die Sulfonamide wurden ohne weitere Aufreinigung in der Stufe B verwendet.

### Stufe A3 - Herstellung der Sulfonamide

Zu einer Lösung aus 27,5 mmol Natriumhydrid (60%ig in Öl) in 25 mL *abs.* THF wird eine Lösung aus 25,0 mmol aromatischem Amin in 35 mL *abs.* THF bei 0 °C unter Rühren und Schutzgasatmosphäre tropfenweise zugegeben. Nach zweistündigem Rühren bei Raumtemperatur wird eine Lösung aus 25,0 mmol des entsprechenden Sulfonylchlorids in 10 mL *abs.* THF bei 0 °C unter Rühren tropfenweise zugegeben. Die Reaktionslösung wird 40 Stunden bei Raumtemperatur gerührt und anschließend mit 100 mL Wasser und 100 mL Dichlormethan versetzt. Die wässrige Phase wird durch Zugabe 5%iger wässriger Natriumhydroxid-Lösung alkalisch gestellt. Die Phasen werden getrennt. Die wässrige Phase wird mit 100 mL Dichlormethan gewaschen und durch Zugabe 25%iger Salzsäure neutral gestellt. Nach mehrmaliger Extraktion mit 100 mL Dichlormethan werden die vereinigten organischen Phasen mit 200 mL Wasser gewaschen und über Magnesiumsulfat getrocknet. Nach Entfernen des Lösungsmittels im Vakuum bleibt das Sulfonamid als Feststoff zurück. Die Sulfonamide wurden ohne weitere Aufreinigung in der Stufe B verwendet.

### Stufe A4 - Herstellung der Sulfonamide

Zu einem Gemisch aus 55 mmol aromatischem Amin und 50 mmol Pyridin werden 50 mmol des entsprechenden Sulfonylchlorids unter Rühren portionsweise zugegeben. Das Gemisch wird kurzzeitig (5-10 min) auf 95-100 °C erwärmt, abgekühlt und mit 100-150 mL Salzsäure (2 mol/L) verrieben. Das ausfallende Sulfonamid wird abfiltriert, mit Wasser neutral gewaschen und getrocknet. Die Sulfonamide wurden ohne weitere Aufreinigung in der Stufe B verwendet.

### Stufe B - Reduktion der Nitro-Gruppe zum primären Amin

Zu einer Lösung aus 8,0 mmol des Produktes aus Stufe A1/A2/A3/A4 in 140 mL Ethylacetat werden unter Rühren 28,0 mmol (Produkte aus Stufe A1) bzw. 56,0 mmol (Produkte aus Stufe A2/A3/A4) SnCl₂·2H₂O bei Raumtemperatur zugegeben. Die Reaktionslösung wird refluxiert. Der Reaktionsverlauf wird mittels Dünnschichtchromatographie verfolgt (Eluenten: Cyclohexan/Ethylacetat 1:1). Nach Beenden der Reaktion (etwa 2-3 h) wird mit 70 mL Ethylacetat verdünnt, mit einer 10%igen wässrigen Kaliumcarbonat-Lösung versetzt und 30 min bei Raumtemperatur gerührt. Die Sn-Verbindungen werden abfiltriert und im Filtrat wird die wässrige von der organischen Phase getrennt. Die organische Phase wird mit 100 ml (2x) einer gesättigten wässrigen Natriumchlorid-Lösung gewaschen und über Magnesiumsulfat getrocknet. Nach Entfernen des Lösungsmittels im Vakuum erfolgt die Aufreinigung durch Umkristallisieren aus Dichlormethan und wenigen Tropfen n-Hexan.

### Stufe C - Herstellung der Harnstoff-Verbindungen

Zu einer Lösung aus 7,0 mmol des Produktes aus Stufe B in Dichlormethan (**I-XVIII)** bzw. Ethylacetat (**XIX**) (20-40 mL) wird eine Lösung aus 14,0 mmol des entsprechenden Isocyanats in 10 mL Dichlormethan **(I-XVIII)** bzw. Ethylacetat (**XIX**) bei Raumtemperatur unter Rühren tropfenweise zugegeben. Die Reaktion wird mittels Dünnschichtchromatographie verfolgt (Eluenten: Cyclohexan/Ethylacetat 1:1). Nach Beenden der Reaktion wird das ausgefallene Produkt abfiltriert, mit Dichlormethan/Ethylacetat gewaschen und im Vakuum getrocknet. In manchen Fällen wird die Reaktionslösung im Vakuum eingeengt und die Kristallisation durch Zugabe weniger Tropfen n-Hexan ausgelöst.

Die Verbindungen **I-II** (Tabelle 2) wurden ausgehend von 2,6-Dinitroanilin hergestellt, das zunächst in 1,2-Diamino-3-nitrobenzol (Reaktionsschema 2, V. Milata, J. Saloň, Org. Prep. Proceed. Int., 31 (3), 347 (1999)) und final nach den allgemeinen Vorschriften der Stufen A1, B und C in das Endprodukt überführt wurde.

Die Verbindungen **III-XVI** (Tabelle 2) wurden ausgehend von 2,4-Dinitroanilin (**III**), 4-Nitro-1,2-phenylendiamin (**IV**), 2,6-Dinitroanilin (**V**) und 2-Nitro-1,4-phenylendiamin **(VI-XVI)** nach den allgemeinen Vorschriften der Stufen A1 **(IV, VI-XVI),** A2 **(III),** A3 **(V),** B **(III-XVI)** und C **(III-XVI)** hergestellt.

Die Verbindungen **XVII-XVIII** (Tabelle 2) wurden ausgehend von 2,4-Dinitrosulfonylchlorid nach den allgemeinen Vorschriften der Stufen A4, B und C hergestellt.

Die Verbindung **XIX** (Tabelle 2) wurde ausgehend von 1,3-Phenylendiamin-Dihydrochlorid hergestellt, das zunächst in 4,6-Diamino-1,3-benzoldisulfonylchlorid (Reaktionsschema 3, G. Barnikow, K. Krüger, G. Hilgetag, Z. Chem., 6 (7), 262 (1966)) und final nach den allgemeinen Vorschriften der Stufen A4 und C in das Endprodukt überführt wurde.

Die Ausgangsverbindungen sind kommerziell erhältlich.

**Tabelle 2: Zusammenstellung ausgewählter Verbindungen der Formel (I)**

| | Ar | Ar¹ | Ar² | l | m | n |
|---|---|---|---|---|---|---|
| **I** | Benzol-1,2,3-triyl | 4-CH₃-C₆H₄ | C₆H₅ | 1 | 0 | 2 |
| **II** | Benzol-1,2,3-triyl | 4-CH₃-C₆H₄ | 4-(CO-CH₃)-C₆H₄ | 1 | 0 | 2 |
| **III** | Benzol-1,2,4-triyl | 4-CH₃-C₆H₄ | C₆H₅ | 1 | 0 | 2 |
| **IV** | Benzol-1,2,5-triyl | 4-CH₃-C₆H₄ | C₆H₅ | 1 | 0 | 2 |
| **V** | Benzol-1,2,6-triyl | 4-CH₃-C₆H₄ | C₆H₅ | 1 | 0 | 2 |
| **VI** | Benzol-1,3,4-triyl | C₆H₅ | C₆H₅ | 1 | 0 | 2 |
| **VII** | Benzol-1,3,4-triyl | 4-CH₃-C₆H₄ | C₆H₅ | 1 | 0 | 2 |
| **VIII** | Benzol-1,3,4-triyl | 4-Cl-C₆H₄ | C₆H₅ | 1 | 0 | 2 |
| **IX** | Benzol-1,3,4-triyl | 4-OCH₃-C₆H₄ | C₆H₅ | 1 | 0 | 2 |
| **X** | Benzol-1,3,4-triyl | 4-CH₃-C₆H₄ | 4-CH₃-C₆H₄ | 1 | 0 | 2 |
| **XI** | Benzol-1,3,4-triyl | 4-CH₃-C₆H₄ | 4-OCH₃-C₆H₄ | 1 | 0 | 2 |
| **XII** | Benzol-1,3,4-triyl | 4-CH₃-C₆H₄ | 4-OC₆H₅-C₆H₄ | 1 | 0 | 2 |
| **XIII** | Benzol-1,3,4-triyl | 4-CH₃-C₆H₄ | 4-Cl-C₆H₄ | 1 | 0 | 2 |
| **XIV** | Benzol-1,3,4-triyl | 4-CH₃-C₆H₄ | 4-(CO-CH₃)-C₆H₄ | 1 | 0 | 2 |
| **XV** | Benzol-1,3,4-triyl | 4-CH₃-C₆H₄ | 2-CF₃-C₆H₄ | 1 | 0 | 2 |
| **XVI** | Benzol-1,3,4-triyl | 4-CH₃-C₆H₄ | CO-C₆H₄ | 1 | 0 | 2 |
| **XVII** | Benzol-1,2,4-triyl | C₆H₅ | C₆H₅ | 0 | 1 | 2 |
| **XVIII** | Benzol-1,2,4-triyl | 4-(CO₂C₂H₅)-C₆H₅ | C₆H₅ | 0 | 1 | 2 |
| **XIX** | Benzol-1,3,4,6-tetryl | C₆H₅ | C₆H₅ | 0 | 2 | 2 |

### Analytische Daten:

### I, C₂₇H₂₅N₅O₄S, M = 515.6, N-(2,3-Bis(3-phenylureido)phenyl)tosylamid

MS (ESI): m/z (%) = 514.0 (76) [M-H]⁻, 395.0 (16) [M-H-Ar²NCO]⁻.
¹H-NMR (500 MHz, DMSO-*d₆*): δ (ppm) = 9.47 (1H, s), 9.34 (1H, s), 9.23 (1H, s), 8.06 (1H, s), 7.80-7.78
(1H, m), 7.70 (1H, s), 7.64-7.63 (2H, m), 7.54-7.52 (2H, m), 7.48-7.46 (2H, m), 7.33-7.25 (6H, m), 7.09-7.05 (1H, m), 7.01-6.98 (1H, m), 6.97-6.94 (1H, m), 6.63-6.61 (1H, m), 2.33 (3H, s).
¹³C-NMR (126 MHz, DMSO-*d*₆): δ (ppm) = 153.66 (NHCONH), 152.61 (NHCONH), 143.14, 139.89, 139.73, 136.92, 136.81, 132.89, 129.53, 128.69, 128.68, 126.76, 125.85, 124.38, 121.80, 121.76, 119.98, 119.17, 118.28, 118.22, 20.95 (CH₃).

### II, C₃₁H₂₉N₅O₆S, M = 599.7, N-(2,3-Bis(3-(4-acetylphenyl)ureido)phenyl)tosylamid

MS (ESI): m/z (%) = 598.1 (100) [M-H]⁻.
¹H-NMR (500 MHz, DMSO-*d*₆): δ (ppm) = 9.74 (1H, s), 9.65 (1H, s), 9.52 (1H, s), 8.14 (1H, s), 7.95-7.94 (2H, m), 7.90-7.88 (2H, m), 7.80-7.78 (2H, m), 7.64-7.62 (4H, m), 7.58-7.57 (2H, m), 7.32-7.31 (2H, m), 7.11-7.08 (1H, m), 6.62-6.61 (1H, m), 2.53 (3H, s), 2.50 (3H, s), 2.33 (3H, s).
¹³C-NMR (126 MHz, DMSO-*d*₆): δ (ppm) = 196.17 (COCH₃), 196.13 (COCH₃), 153.19 (NHCONH), 152.20 (NHCONH), 144.50, 144.28, 143.14, 136.81, 136.61, 133.13, 130.46, 130.41, 129.52, 129.52, 129.50, 126.75, 126.19, 124.13, 120.00, 119.42, 117.16, 117.16, 26.21 (CH₃), 26.18 (CH₃), 20.91 (CH₃).

### III, C₂₇H₂₅N₅O₄S, M = 515.6, N-(2,4-Bis(3-phenylureido)phenyl)tosylamid

MS (ESI): m/z (%) = 514.1 (100) [M-H]⁻.
¹H-NMR (500 MHz, DMSO-*d*₆): δ (ppm) = 9.54 (1H, s), 9.27 (1H, s), 8.78 (1H, s), 8.53 (1H, s), 8.28 (1H, s), 8.15 (1H, d, *J* = 2.2 Hz), 7.61-7.60 (2H, m), 7.53-7.52 (2H, m), 7.46-7.44 (2H, m), 7.36-7.34 (2H, m), 7.33-7.30 (2H, m), 7.29-7.26 (2H, m), 7.08 (1H, dd, 8.7, 2.2 Hz), 7.01-6.95 (2H, m), 6.39 (1H, d, 8.6 Hz), 2.34 (3H, s). ¹³C-NMR (126 MHz, DMSO-*d*₆): δ (ppm) = 152.35 (NHCONH), 152.31 (NHCONH), 143.16, 139.84, 139.54, 139.23, 137.78, 136.48, 129.43, 128.78, 128.74, 128.15, 127.26, 121.87, 121.84, 118.38, 118.19, 118.17, 111.23, 109.79, 20.97 (CH₃).

### IV, C₂₇H₂₅N₅O₄S, M = 515.6, N-(2,5-Bis(3-phenylureido)phenyl)tosylamid

MS (ESI): m/z (%) = 514.0 (100) [M-H]⁻.
¹H-NMR (500 MHz, DMSO-*d*₆): δ (ppm) = 9.47 (1H, s), 9.23 (1H, s), 8.54 (1H, s), 8.49 (1H, s), 8.06 (1H, s), 7.73 (1H, d, *J* = 8.7 Hz), 7.65-7.63 (2H, m), 7.50-7.48 (2H, m), 7.45-7.43 (2H, m), 7.34-7.26 (7H, m), 7.01 (1H, d, *J* = 2.2 Hz), 6.99-6.95 (2H, m), 2.31 (3H, s).
¹³C-NMR (126 MHz, DMSO-*d*₆): δ (ppm) = 152.84 (NHCONH), 152.25 (NHCONH), 143.15, 139.92, 139.65, 136.60, 134.86, 129.68, 129.49, 128.73, 128.72, 127.01, 126.78, 122.72, 121.76, 121.70, 118.16, 118.12, 117.01, 116.51, 20.97 (CH₃).

### V, C₂₇H₂₅N₅O₄S, M = 515.6, N-(2,6-Bis(3-phenylureido)phenyl)tosylamid

MS (ESI): m/z (%) = 514.1 (100) [M-H]⁻.
¹H-NMR (500 MHz, DMSO-*d*₆): δ (ppm) = 8.92 (2H, s), 8.90 (1H, s), 7.96 (2H, s), 7.44-7.41 (8H, m), 7.31-7.28 (4H, m), 7.22-7.18 (1H, m), 7.13-7.11 (2H, m), 7.00-6.97 (2H, m), 2.00 (3H, s).
¹³C-NMR (126 MHz, DMSO-*d*₆): δ (ppm) = 152.59 (NHCONH), 143.70, 139.66, 137.95, 136.65, 129.51, 128.98, 128.40, 126.90, 122.26, 118.59, 116.47, 116.36, 20.96 (CH₃).

### VI, C₂₆H₂₃N₅O₄S, M = 501.6, N-(3,4-Bis(3-phenylureido)phenyl)benzolsulfonamid

MS (ESI): m/z (%) = 500.1 (100) [M-H]⁻.
¹H-NMR (500 MHz, DMSO-*d*₆): δ (ppm) = 10.24 (1H, s), 9.11 (1H, s), 8.96 (1H, s), 8.09 (1H, s), 7.89 (1H, s), 7.85-7.84 (2H, m), 7.65-7.55 (4H, m), 7.50-7.47 (4H, m), 7.36 (1H, d, *J* = 8.6 Hz), 7.31-7.25 (4H, m), 7.00-6.94 (2H, m), 6.85 (1H, dd, *J* = 8.4, 1.4 Hz).
¹³C-NMR (126 MHz, DMSO-*d*₆): δ (ppm) = 153.41 (NHCONH), 152.79 (NHCONH), 139.86, 139.73, 139.71, 134.15, 132.99, 132.82, 129.21, 128.83, 128.76, 126.75, 126.40, 125.54, 121.92, 121.76, 118.22, 118.14, 115.35, 114.87.

### VII, C₂₇H₂₅N₅O₄S, M = 515.6, N-(3,4-Bis(3-phenylureido)phenyl)tosylamid

MS (ESI): m/z (%) = 514.1 (88) [M-H]⁻.
¹H-NMR (500 MHz, DMSO-*d*₆): δ (ppm) = 10.16 (1H, s), 9.11 (1H, s), 8.96 (1H, s), 8.09 (1H, s), 7.88 (1H, s), 7.73-7.72 (2H, m), 7.63 (1H, d, *J* = 2.4 Hz), 7.50-7.46 (4H, m), 7.36-7.33 (3H, m), 7.31-7.25 (4H, m), 7.00-6.94 (2H, m), 6.84 (1H, dd, *J* = 8.7, 2.5 Hz), 2.34 (3H, s).
¹³C-NMR (126 MHz, DMSO-*d*₆): δ (ppm) = 153.42 (NHCONH), 152.79 (NHCONH), 143.17, 139.87, 139.72, 136.86, 134.34, 133.00, 129.66, 128.83, 128.76, 126.81, 126.23, 125.57, 121.91, 121.75, 118.22, 118.13, 115.13, 114.64, 20.98 (CH₃).

### VIII, C₂₆H₂₂ClN₅O₄S, M = 536.0, N-(3,4-Bis(3-phenylureido)phenyl)-4-chlorbenzol-sulfonamid

MS (ESI): m/z (%) = 534.1 (100) [M-H]⁻.
¹H-NMR (500 MHz, DMSO-*d*₆): δ (ppm) = 10.28 (1H, s), 9.10 (1H, s), 8.95 (1H, s), 8.09 (1H, s), 7.90 (1H, s), 7.84-7.82 (2H, m), 7.65-7.63 (2H, m), 7.62 (1H, d, *J* = 2.5 Hz), 7.50-7.47 (4H, m), 7.39 (1H, d, *J* = 8.7 Hz), 7.31-7.25 (4H, m), 6.99-6.94 (2H, m), 6.84 (1H, dd, *J* = 8.7, 2.5 Hz).
¹³C-NMR (126 MHz, DMSO-*d*₆): δ (ppm) = 153.36 (NHCONH), 152.77 (NHCONH), 139.82, 139.68, 138.50, 137.76, 133.71, 132.95, 129.37, 128.78, 128.72, 128.67, 126.70, 125.48, 121.89, 121.75, 118.22, 118.15, 115.72, 115.23.

### IX, C₂₇H₂₅N₅O₅S, M = 531.6, N-(3,4-Bis(3-phenylureido)phenyl)-4-methoxybenzol-sulfonamid

MS (ESI): m/z (%) = 530.1 (100) [M-H]⁻.
¹H-NMR (500 MHz, DMSO-*d*₆): δ (ppm) = 10.08 (1H, s), 9.10 (1H, s), 8.96 (1H, s), 8.08 (1H, s), 7.88 (1H, s), 7.78-7.76 (2H, m), 7.62-7.62 (1H, m), 7.50-7.46 (4H, m), 7.35 (1H, d, *J* = 8.6 Hz), 7.31-7.25 (4H, m), 7.08-7.07 (2H, m), 7.00-6.94 (2H, m), 6.85-6.83 (1H, m), 3.80 (3H, s).
¹³C-NMR (126 MHz, DMSO-*d*₆): δ (ppm) = 162.40, 153.42 (NHCONH), 152.79 (NHCONH), 139.87, 139.72, 134.44, 132.97, 131.35, 128.96, 128.82, 128.75, 126.19, 125.54, 121.90, 121.74, 118.21, 118.13, 115.14, 114.66, 114.35, 55.59 (OCH₃).

### X, C₂₉H₂₉N₅O₄S, M = 543.6, N-(3,4-Bis(3-(4-tolyl)ureido)phenyl)tosylamid

MS (ESI): m/z (%) = 542.2 (38) [M-H]⁻.
¹H-NMR (500 MHz, DMSO-*d*₆): δ (ppm) = 10.13 (1H, s), 8.99 (1H, s), 8.84 (1H, s), 8.03 (1H, s), 7.82 (1H, s), 7.72-7.70 (2H, m), 7.61 (1H, d, *J* = 2.2 Hz), 7.37-7.30 (7H, m), 7.11-7.06 (4H, m), 6.81 (1H, dd, *J* = 8.7, 2.2 Hz), 2.34 (3H, s), 2.24 (3H, s), 2.23 (3H, s).
¹³C-NMR (126 MHz, DMSO-*d*₆): δ (ppm) = 153.43 (NHCONH), 152.80 (NHCONH), 143.14, 137.29, 137.13, 136.86, 134.19, 132.97, 130.72, 130.52, 129.64, 129.21, 129.15, 126.80, 126.27, 125.44, 118.31, 118.22, 115.05, 114.62, 20.97 (CH₃), 20.34 (CH₃), 20.33 (CH₃).

### XI, C₂₉H₂₉N₅O₆S, M = 575.6, N-(3,4-Bis(3-(4-methoxyphenyl)ureido)phenyl)tosyl-amid

MS (ESI): m/z (%) = 574.2 (80) [M-H]⁻.
¹H-NMR (500 MHz, DMSO-*d*₆): δ (ppm) = 10.12 (1H, s), 8.91 (1H, s), 8.76 (1H, s), 8.00 (1H, s), 7.79 (1H, s), 7.72-7.70 (2H, m), 7.60 (1H, d, *J* = 2.5 Hz), 7.39-7.30 (7H, m), 6.90-6.84 (4H, m), 6.81 (1H, dd, J = 8.7, 2.5 Hz), 3.71 (3H, s), 3.70 (3H, s), 2.34 (3H, s).
¹³C-NMR (126 MHz, DMSO-*d*₆): δ (ppm) = 154.52 (NHCONH), 154.40 (NHCONH), 153.59, 152.97, 143.14, 136.87, 134.15, 133.06, 132.91, 132.74, 129.64, 126.80, 126.40, 125.43, 120.03, 119.94, 115.02, 114.65, 114.03, 113.96, 55.16 (OCH₃), 55.13 (OCH₃), 20.97 (CH₃).

### XII, C₃₉H₃₃N₅O₆S, M = 699.8, N-(3,4-Bis(3-(4-phenoxyphenyl)ureido)phenyl)tosyl-amid

MS (ESI): m/z (%) = 700.2 (100) [M+H]⁺, 515.1 (63) [M+H-Ar²NH₂]⁺, 489.2 (43) [M+H-Ar²NH₂-Ar²NCO]⁺.
¹H-NMR (500 MHz, DMSO-*d*₆): δ (ppm) = 10.14 (1H, s), 9.12 (1H, s), 8.98 (1H, s), 8.07 (1H, s), 7.87 (1H, s), 7.73-7.72 (2H, m), 7.62 (1H, d, *J* = 2.4 Hz), 7.52-7.48 (4H, m), 7.38-7.33 (7H, m), 7.10-7.06 (2H, m), 7.01-6.94 (8H, m), 6.84 (1H, dd, *J* = 8.7, 2.5 Hz), 2.34 (3H, s).
¹³C-NMR (126 MHz, DMSO-*d*₆): δ (ppm) = 157.61, 157.58, 153.47 (NHCONH), 152.86 (NHCONH), 150.77, 150.60, 143.09, 136.87, 135.84, 135.65, 134.32, 132.99, 129.85, 129.83, 129.59, 126.76, 126.30, 125.53, 122.75, 122.70, 119.94, 119.86, 119.69, 119.67, 117.65, 117.57, 115.16, 114.69, 20.93 (CH₃).

### XIII, C₂₇H₂₃Cl₂N₅O₄S, M = 584.5, N-(3,4-Bis(3-(4-chlorphenyl)ureido)phenyl)tosyl-amid

MS (ESI): m/z (%) = 582.1 (54) [M-H]⁻.
¹H-NMR (500 MHz, DMSO-*d*₆): δ (ppm) = 10.16 (1H, s), 9.24 (1H, s), 9.11 (1H, s), 8.10 (1H, s), 7.89 (1H, s), 7.72-7.70 (2H, m), 7.60 (1H, d, *J* = 2.2 Hz), 7.51-7.48 (4H, m), 7.35-7.29 (7H, m), 6.83 (1H, dd, *J* = 8.7, 2.3 Hz), 2.34 (3H, s). ¹³C-NMR (126 MHz, DMSO-*d*₆): δ (ppm) = 153.30 (NHCONH), 152.67 (NHCONH), 143.17, 138.86, 138.69, 136.82, 134.51, 132.93, 129.64, 128.65, 128.58, 126.79, 126.09, 125.74, 125.45, 125.27, 119.72, 119.64, 115.23, 114.62, 20.97 (CH₃).

### XIV, C₃₁H₂₉N₅O₆S, M = 599.7, N-(3,4-Bis(3-(4-acetylphenyl)ureido)phenyl)tosyl-amid

MS (ESI): m/z (%) = 598.1 (82) [M-H]⁻, 463.1 (23) [M-H-Ar²NH₂]⁻, 302.0 (11) [M-H-Ar²NH₂-Ar²NCO]⁻.
¹H-NMR (500 MHz, DMSO-*d*₆): δ (ppm) = 10.21 (1H, s), 9.53 (1H, s), 9.40 (1H, s), 8.22 (1H, s), 8.01 (1H, s), 7.93-7.91 (2H, m), 7.91-7.89 (2H, m), 7.73-7.71 (2H, m), 7.64 (1H, d, *J* = 2.3 Hz), 7.62-7.58 (4H, m), 7.37-7.34 (3H, m), 6.86 (1H, dd, *J* = 8.7, 2.3 Hz), 2.52 (3H, s), 2.50 (3H, s), 2.34 (3H, s).
¹³C-NMR (126 MHz, DMSO-*d*₆): δ (ppm) = 196.28, 196.26, 153.09 (NHCONH), 152.44 (NHCONH), 144.49, 144.31, 143.23, 136.80, 134.75, 132.88, 130.54, 130.39, 129.69, 129.69, 129.65, 126.82, 125.94, 125.89, 117.19, 117.11, 115.35, 114.60, 26.33 (CH₃), 26.31 (CH₃), 20.99 (CH₃).

### XV, C₂₉H₂₃F₆N₅O₄S, M = 651.6, N-(3,4-Bis(3-(2-(trifluormethyl)phenyl)ureido)phenyl)tosylamid

MS (ESI): m/z (%) = 650.1 (100) [M-H]⁻, 489.1 (20) [M-H-Ar²NH₂]⁻, 302.1 (16) [M-H-Ar²NH₂-Ar²NCO]⁻.
¹H-NMR (500 MHz, DMSO-*d*₆): δ (ppm) = 10.15 (1H, s), 8.77 (1H, s), 8.66 (1H, s), 8.46 (1H, s), 8.26 (1H, s), 7.98-7.92 (2H, m), 7.71-7.57 (7H, m), 7.37-7.33 (3H, m), 7.30-7.27 (1H, m), 7.25-7.22 (1H, m), 6.85 (1H, dd, *J* = 8.7, 2.3 Hz), 2.33 (3H, s).
¹³C-NMR (126 MHz, DMSO-*d*₆): δ (ppm) = 153.39 (NHCONH), 153.00 (NHCONH), 143.17, 136.80, 136.59, 136.35, 134.41, 132.81, 132.54, 129.62, 126.77, 126.28, 126.09, 125.92, 125.40, 125.07, 123.88, 123.49, 122.90, 120.31, 119.65, 115.30, 20.93 (CH₃).

### XVI, C₂₉H₂₅N₅O₆S, M = 571.6, N,N'-(((4-Tosylamido-1,2-phenylen)bis(azandiyl)) bis(carbonyl))dibenzamid

MS (ESI): m/z (%) = 570.1 (25) [M-H]⁻, 449.0 (100) [M-H-Ar²NH₂]⁻, 302.0 (63) [M-H-Ar²NH₂-Ar²NCO]⁻.
¹H-NMR (500 MHz, DMSO-*d*₆): δ (ppm) = 11.09 (1H, s), 11.08 (1H, s), 10.85 (1H, s), 10.45 (1H, s), 10.37 (1H, s), 8.01-7.99 (2H, m), 7.96-7.94 (2H, m), 7.83 (1H, d, *J* = 2.5 Hz), 7.77-7.75 (2H, m), 7.65-7.60 (2H, m), 7.53-7.46 (5H, m), 7.37-7.36 (2H, m), 6.97 (1H, dd, *J* = 8.7, 2.5 Hz), 2.34 (3H, s).
¹³C-NMR (126 MHz, DMSO-*d*₆): δ (ppm) = 168.70, 168.64, 152.23 (NHCONH), 151.43 (NHCONH), 143.30, 136.79, 135.88, 132.97, 132.90, 132.57, 132.39, 132.21, 129.69, 128.47, 128.47, 128.24, 128.23, 126.81, 126.44, 124.87, 115.83, 114.38, 20.96 (CH₃).

### XVII, C₂₆H₂₃N₅O₄S, M = 501.6, N-Phenyl-2,4-bis(3-phenylureido)benzolsulfonamid

MS (ESI): m/z (%) = 500.1 (100) [M-H]⁻, 381.1 (22) [M-H-Ar²NCO]⁻.
¹H-NMR (500 MHz, DMSO-*d*₆): δ (ppm) = 10.28 (1H, s), 9.65 (1H, s), 9.15 (1H, s), 8.67 (1H, s), 8.48 (1H, s), 8.15 (1H, d, *J* = 1.2 Hz), 7.67 (1H, d, *J* = 8.8 Hz), 7.56-7.54 (2H, m), 7.47-7.46 (2H, m), 7.37-7.27 (5H, m), 7.24-7.21 (2H, m), 7.13-7.12 (2H, m), 7.03-6.98 (3H, m).
¹³C-NMR (126 MHz, DMSO-*d*₆): δ (ppm) = 152.04 (NHCONH), 151.75 (NHCONH), 144.48, 139.68, 139.18, 138.16, 137.24, 130.32, 129.13, 128.81, 128.77, 124.34, 122.28, 122.16, 120.67, 119.07, 118.54, 118.47, 111.07, 110.68.

### XVIII, C₂₉H₂₇N₅O₆S, M = 573.6, Ethyl 4-(2,4-bis(3-phenylureido)phenylsulfonamido) benzoat

MS (ESI): m/z (%) = 572.1 (100) [M-H]⁻.
¹H-NMR (500 MHz, DMSO-*d*₆): δ (ppm) = 10.77 (1H, s), 9.60 (1H, s), 9.15 (1H, s), 8.66 (1H, s), 8.38 (1H, s), 8.07 (1H, d, *J* = 2.1 Hz), 7.81-7.80 (2H, m), 7.74 (1H, d, *J* = 8.9 Hz), 7.50-7.48 (2H, m), 7.45-7.43 (2H, m), 7.37 (1H, dd, *J* = 8.9, 2.1 Hz), 7.32-7.26 (4H, m), 7.24-7.22 (2H, m), 7.02-6.97 (2H, m), 4.19 (2H, q, *J* = 7.1 Hz), 1.24 (3H, t, *J* = 7.1 Hz).
¹³C-NMR (126 MHz, DMSO-*d*₆): δ (ppm) = 165.02, 151.95 (NHCONH), 151.59 (NHCONH), 144.72, 141.88, 139.54, 139.11, 138.09, 130.46, 130.33, 128.77, 128.70, 124.94, 122.26, 122.10, 118.99, 118.82, 118.43, 118.43, 111.23, 111.01, 60.41 (CH₂), 14.08 (CH₃).

### XIX, C₃₂H₂₈N₆O₆S₂, M = 656.7, N¹,N³-Diphenyl-4,6-bis(3-phenylureido)benzol-1,3-disulfonamid

MS (ESI): m/z (%) = 655.1 (100) [M-H]⁻, 536.1 (18) [M-H-Ar²NCO]⁻.
¹H-NMR (500 MHz, DMSO-*d*₆): δ (ppm) = 10.47 (2H, s), 9.75 (2H, s), 8.85 (1H, s), 8.52 (2H, s), 8.15 (1H, s), 7.48-7.47 (4H, m), 7.32-7.29 (4H, m), 7.21-7.18 (4H, m), 7.04-7.02 (8H, m).
¹³C-NMR (126 MHz, DMSO-*d*₆): δ (ppm) = 150.92 (NHCONH), 141.38, 139.07, 136.38, 131.09, 129.19, 128.70, 124.80, 122.50, 121.03, 118.82, 118.75, 113.36.

Der Auftrag einer wässrigen Auftragssuspension zur Ausbildung der wärmeempfindlichen farbbildenden Schicht eines wärmeempfindlichen Aufzeichnungspapiers erfolgte im Labormaßstab mittels einer Stabrakel auf eine Seite eines synthetischen Basispapieres (Yupo® FP680) von 63 g/m². Nach Trocknung wurde ein thermisches Aufzeichnungsblatt erhalten. Die Auftragsmenge der wärmeempfindlichen farbbildenden Schicht lag zwischen 3,8 und 4,2 g/m².

Anhand der vorstehend gemachten Angaben wurde ein wärmeempfindliches Aufzeichnungsmaterial bzw. Thermopapier hergestellt, wobei die folgenden Rezepturen wässriger Auftragssuspensionen zur Ausbildung eines Verbundgebildes auf einem Trägersubstrat herangezogen und anschließend in üblicher Weise die weiteren Schichten, insbesondere eine Schutzschicht, ausgebildet wurden, worauf hier nicht gesondert eingegangen werden soll.

Herstellen der Dispersionen (jeweils für 1 Gew.-Teil) für die Auftragssuspensionen
Eine wässrige **Dispersion A** (Farbbildnerdispersion) wird hergestellt durch Mahlen von 20 Gew.-Teilen 3-*N*-n-Dibutylamin-6-methyl-7-anilinofluoran (ODB-2) mit 33 Gew.-Teilen einer 15%igen wässrigen Lösung von Ghosenex™ L-3266 (sulfonierter Polyvinylalkohol, Nippon Ghosei) in einer Perlen-Mühle.
Eine wässrige **Dispersion B** (Farbentwicklerdispersion) wird durch Mahlen von 40 Gew.-Teilen des Farbentwicklers zusammen mit 66 Gew.-Teilen einer 15%igen wässrigen Lösung von Ghosenex™ L-3266 in der Perlen-Mühle hergestellt.
Eine wässrige **Dispersion C** (Sensibilisierungsmittel-Dispersion) wird hergestellt durch Mahlen von 40 Gew.-Teilen Sensibilisierungsmittel mit 33 Gew.-Teilen einer 15%igen wässrigen Lösung von Ghosenex™ L-3266 in einer Perlen-Mühle.
   Alle durch Mahlen erzeugten Dispersionen haben eine mittlere Körngröße D_{(4,3)} von 0,80 bis 1,20 µm. Die Messung der Korngrößenverteilung der Dispersionen erfolgte durch Laserbeugung mit einem Coulter LS230-Gerät der Fa. Beckman Coulter.
Die **Dispersion D** (Gleitmitteldispersion) ist eine 20%ige Zinkstearat-Dispersion, bestehend aus 9 Gew.-Teilen Zn-Stearat, 1 Gew.-Teil Ghosenex™ L-3266 und 40 Teilen Wasser.

Pigment **P** ist eine 72%ige Streichkaolin-Suspension (Lustra® S, BASF).

Der **Binder** besteht aus einer 10%igen wässrigen Polyvinylalkohollösung (Mowiol 28-99, Kuraray Europe).

Die wärmeempfindliche Auftragssuspension wird durch Mischen unter Rühren von 1 Teil **A,** 1 Teil **B,** 1 Teil **C,** 56 Teilen **D,** 146 Teilen Pigment **P** und 138 Teilen **Binder**-Lösung (alles Gew.-Teile) unter Berücksichtigung der Eintragsreihenfolge **B, D, C, P, A, Binder** hergestellt und mit Wasser auf einen Feststoffgehalt von etwa 25% gebracht.

Die so erhaltenen wärmeempfindlichen Beschichtungssuspensionen wurden herangezogen, um Verbundgebilde aus Papierträger und Thermoreaktionsschicht herzustellen.

Die thermischen Aufzeichnungsmaterialien wurden wie nachstehend ausgewertet (Tabellen 3, 4 und 5).

### (1) Dynamische Farbdichte:

Die Papiere (6 cm breite Streifen) wurden thermisch unter Verwendung des Atlantek 200 Testdruckers (Fa. Atlantek, USA) mit einer Kyocera-Druckleiste von 200 dpi und 560 Ohm bei einer angelegten Spannung von 20,6 V und einer maximalen Pulsbreite von 0,8 ms mit einem Schachbrett-Muster mit 10 Energieabstufungen bedruckt. Die Bilddichte (optische Dichte, o.D.) wurde mit einem Macbeth-Densitometer RD-914 von Gretag bei einer Energiestufe von 0,45 mJ/Dot gemessen. Die Messunsicherheit der o.D.-Werte wird mit ≤2% veranschlagt.

### (2) Statische Farbdichte (Starttemperatur):

Das Aufzeichnungsblatt wurde gegen eine Reihe auf unterschiedlichen Temperaturen erhitzten und thermostatierten metallischen Stempeln mit einem Anpressdruck von 0,2 kg/cm² und einer Kontaktzeit von 5 sec. gepresst (Thermoprüfer TP 3000QM, Maschinenfabrik Hans Rychiger AG, Steffisburg, Schweiz). Die Bilddichte (opt. Dichte) der so erzeugten Bilder wurde mit einem Macbeth-Densitometer RD-914 von Gretag gemessen. Der statische Startpunkt ist definitionsmäßig die niedrigste Temperatur, bei welcher eine optische Dichte von 0,2 erreicht wird. Die Genauigkeit des Messverfahrens beträgt ≤±0,5 °C.

### (3) Beständigkeitsprüfung des Druckbildes:

### a) Beständigkeit des Druckbildes unter den Bedingungen der künstlichen Alterung:

Je eine gemäß dem Verfahren von (1) dynamisch aufgezeichnete Probe des thermischen Aufzeichnungspapiers wurde für 7 Tage unter folgenden Bedingungen gelagert:
i) 50 °C (Trocken-Alterung),
ii) 40 °C, 85% relative Feuchte (Feucht-Alterung) und
iii) unter Kunstlicht von Leuchtstoffröhren, Beleuchtungsstärke 16000 Lux (Licht-Alterung)

Nach Ablauf der Testzeit wurde die Bilddichte bei einer Bestromungsenergie von 0,45 mJ/dot gemessen und entsprechend der Formel (Gl. 1) in Bezug zu den entsprechenden Bilddichtewerten vor der künstlichen Alterung gesetzt.

### b) Weichmacherbeständigkeit:

Auf die gemäß dem Verfahren von (1) dynamisch aufgezeichnete Probe des thermischen Aufzeichnungspapiers wurde eine weichmacherhaltige Frischhaltefolie (PVC-Folie mit 20-25% Dioctyladipat) unter Vermeiden von Falten und Lufteinschlüssen in Kontakt gebracht, zu einer Rolle gewickelt und 16 Stdn. gelagert. Eine Probe wurde bei Raumtemperatur (20-22 °C), eine zweite bei 40 °C gelagert. Nach Abziehen der Folie wurde die Bilddichte (o.D.) gemessen und entsprechend der Formel (Gl. 1) in Bezug zu den entsprechenden Bilddichtewerten vor der Weichmacher-Einwirkung gesetzt.

### c) Beständigkeit gegenüber Haftkleber:

Auf die gemäß dem Verfahren von (1) dynamisch aufgezeichnete Probe des thermischen Aufzeichnungspapiers wurde je ein Streifen transparentes Tesa-Selbstklebeband (tesafilm® kristall-klar, #57315) und getrennt davon ein Streifen Tesa-Verpackungsklebeband (#04204) unter Vermeiden von Falten und Lufteinschlüssen geklebt. Nach Lagerung bei Raumtemperatur (20-22°C) wurde nach 24 Stunden und nach 7 Tagen die Bilddichte (o.D.) -durch das jeweilige Klebeband hindurch- gemessen und entsprechend der Formel (Gl. 1) in Bezug zu den analog bestimmten Bilddichtewerten der frisch beklebten Muster gesetzt. $% verbleibende Bilddichte = \left(\frac{Bilddichte nach Test}{Bilddichte vor Test}\right) ∗ 100$ (Gl. 1)

Die Streuung der nach (Gl. 1) berechneten %-Werte beträgt ≤±2 Prozentpunkte.

### 4) Lagerfähigkeit des unbedruckten Thermopapieres:

Ein Blatt Aufzeichnungspapier wurde in drei identische Streifen geschnitten. Ein Streifen wurde gemäß dem Verfahren von (1) dynamisch aufgezeichnet und die Bilddichte bestimmt. Die beiden anderen Streifen wurden in unbedrucktem (weißen) Zustand für 4 Wochen in einem Klima von a) 40 °C und 85% relativer Feuchte (r. F.) und b) 60 °C und 50% relativer Feuchte (r. F.) gelagert.

Nach Klimatisierung der Papiere bei Raumtemperatur wurden diese gemäß dem Verfahren von (1) dynamisch bedruckt und die Bilddichte bei einer Bestromungsenergie von 0,45 mJ/dot mit dem Densitometer bestimmt. Die verbleibende Schreibleistung (%) der gelagerten zu den frischen (nicht gealterten) Mustern wurde gemäß der Gleichung (Gl. 1) berechnet.

Die Tabellen 3 bis 5 fassen die Auswertung der gefertigten Aufzeichnungsmaterialien zusammen.

**Tabelle 3: Bilddichte, Starttemperatur und künstliche Alterung**

| Fa rbentwickler | o.D. (0,45 mJ/dot) | Startpunkt (°C) | Künstliche Alterung* | | |
|---|---|---|---|---|---|
| | | | trocken | feucht | Licht |
| **III** | 1,19 | 79 | 97 | 98 | 86 |
| **IV** | 1,17 | 82 | 96 | 98 | 80 |
| **XIV** | 1,18 | 85 | 100 | 98 | 87 |
| **XVII** | 1,19 | 82 | 97 | 100 | 74 |
| **XVIII** | 1,22 | 81 | 98 | 100 | 76 |
| **XIX** | 1,29 | 80 | 98 | 99 | 80 |
| **Y** | 1,23 | 82 | 100 | 98 | 72 |
| **Z** | 1,25 | 84 | 99 | 98 | 80 |
| **PF201** | 1,23 | 76 | 100 | 96 | 82 |

| | | | | | |
|---|---|---|---|---|---|
| *Prozentuale verbleibende Bilddichte entsprechend Gl. 1 | | | | | |

**Tabelle 4: Beständigkeit des Druckbildes**

| Fa rbentwickler | Tesa-Klebeband* | | | | Weichmacher-Folie* | |
|---|---|---|---|---|---|---|
| | 24 h | | 7 Tage | | 16 h | |
| | #57315 | #04204 | #57315 | #04204 | R.T. | 40 °C |
| **III** | 86 | 80 | 65 | 38 | 96 | 75 |
| **IV** | 92 | 89 | 73 | 60 | 95 | 71 |
| **XIV** | 90 | 95 | 75 | 79 | 99 | 91 |
| **XVII** | 78 | 54 | 50 | 14 | 96 | 78 |
| **XVIII** | 80 | 66 | 51 | 30 | 97 | 83 |
| **XIX** | 81 | 61 | 58 | 21 | 99 | 80 |
| **Y** | 32 | 11 | 9 | 7 | 67 | 7 |
| **Z** | 54 | 31 | 13 | 16 | 88 | 32 |
| **PF201** | 71 | 43 | 29 | 11 | 96 | 68 |

| | | | | | | |
|---|---|---|---|---|---|---|
| *Prozentuale verbleibende Bilddichte entsprechend Gl. 1 | | | | | | |

**Tabelle 5: Schreibleistung nach Lagerung**

| Farbentwickler | o.D. vor Lagerung | 4 Wochen 40 °C / 85% r. F. | | 4 Wochen 60 °C / 50% r. F. | |
|---|---|---|---|---|---|
| | | o.D. nach Lagerung | verbleibende o.D. (%) | o.D. nach Lagerung | verbleibende o.D. (%) |
| **III** | 1,19 | 1,18 | 99 | 1,05 | 88 |
| **IV** | 1,17 | 1,12 | 96 | 1,05 | 90 |
| **XIV** | 1,18 | 1,16 | 98 | 1,01 | 86 |
| **XVII** | 1,19 | 1,18 | 99 | 1,02 | 86 |
| **XVIII** | 1,22 | 1,16 | 95 | 1,04 | 85 |
| **XIX** | 1,29 | 1,29 | 100 | 1,11 | 86 |
| **Y** | 1,23 | - | - | 1,05 | 85 |
| **Z** | 1,25 | 1,25 | 100 | 1,11 | 89 |
| **PF201** | 1,23 | 1,19 | 97 | 0,74 | 60 |

Aus vorstehenden Beispielen lässt sich entnehmen, dass das wärmeempfindliche Aufzeichnungsmaterial der vorliegenden Erfindung insbesondere die folgenden vorteilhaften Eigenschaften zeigt:
(1) Das aufgezeichnete Bild der wärmeempfindlichen Papiere mit den erfindungsgemäßen Farbentwicklern weist eine Druckdichte (optische Dichte) auf, die vergleichbar jener der Entwickler der Vergleichsmuster ist (Tabelle 3).
(2) Die Temperatur, ab welcher eine visuell merkliche Vergrauung der erfindungsgemäßen Papiere eintritt (statischer Startpunkt), ist vergleichbar oder höher als bei den Vergleichspapieren und erfüllt die Anforderungen an markttaugliche wärmeempfindliche Aufzeichnungsmaterialien (Tabelle 3).
(3) Die dem Alterungs-Test unterworfenen Papiere offenbaren eine hohe Bildbeständigkeit. Diese ist besser oder vergleichbar zu den Vergleichspapieren (Tabelle 3).
(4) Das Druckbild ist nach Einwirken hydrophober Agenzien (Klebstoffe, Weichmacher) praktisch nicht verblasst. Die Bildbeständigkeit ist besser oder vergleichbar der Leistung der bekannten nicht-phenolischen Farbentwickler (Tabelle 4).
(5) Das Bedrucken der über mehrere Wochen unter extremen Bedingungen gelagerten Aufzeichnungsmaterialien führt zu Bilddichten, welche nahezu identisch jenen der ungelagerten (frischen) Papiere sind (Tabelle 5).
(6) Mit den erfindungsgemäßen Farbentwicklern lässt sich ein in allen wichtigen anwendungstechnischen Belangen hochwertiges wärmeempfindliches Aufzeichnungsmaterial erhalten. Kein mit Farbentwicklern des Standes der Technik erhaltenes wärmeempfindliches Aufzeichnungsmaterial weist ein vergleichbar ausgewogenes Leistungsprofil über alle Eigenschaften auf.
(7) Der Vergleich des wärmeempfindlichen Aufzeichnungsmaterials, enthaltend den Farbentwickler **Y,** mit wärmeempfindlichen Aufzeichnungsmaterialien, enthaltend die Farbentwickler **XVII** und **XIX,** sowie des wärmeempfindlichen Aufzeichnungsmaterials, enthaltend den Farbentwickler **Z,** mit wärmeempfindlichen Aufzeichnungsmaterialien, enthaltend die Farbentwickler **III** und **IV,** verdeutlicht die Steigerung der Bildbeständigkeit, welche, bei ansonsten vergleichbarer Chemie, der Erhöhung der Dichte der am Farbbildungs- und Stabilisierungsprozess beteiligten funktionellen Gruppen geschuldet ist (Tabelle 4).

## Patentansprüche

1. Verbindung der Formel (I),
Ar(NHSO₂Ar¹)ₗ(SO₂NHAr¹)ₘ(NHC(O)NHAr²)ₙ (I),
wobei
l und m unabhängig voneinander 0, 1, 2, 3 und/oder 4 sind und die Summe l+m gleich oder größer als 1 ist,
n 2, 3, 4 oder 5 ist,
Ar ein (l+m+n)-fach substituierter Benzol-Rest ist,
Ar¹ ein unsubstituierter oder substituierter aromatischer Rest ist und
Ar² ein unsubstituierter oder substituierter Phenyl-Rest oder ein Benzoyl-Rest ist.

2. Verbindung nach Anspruch 1, wobei l 0 oder 1 ist.

3. Verbindung nach mindestens einem der vorangegangenen Ansprüche, wobei m 0, 1 oder 2 ist.

4. Verbindung nach mindestens einem der vorangegangenen Ansprüche, wobei n 2 ist.

5. Verbindung nach mindestens einem der vorangegangenen Ansprüche, wobei l 1, m 0 und n 2 ist.

6. Verbindung nach mindestens einem der vorangegangenen Ansprüche, wobei l 0, m 1 und n 2 ist.

7. Verbindung nach mindestens einem der vorangegangenen Ansprüche, wobei l 0, m 2 und n 2 ist.

8. Verbindung nach mindestens einem der vorangegangenen Ansprüche, wobei Ar ein 3- oder 4-fach substituierter Benzol-Rest, vorzugsweise ein 3-fach substituierter Benzol-Rest, ist.

9. Verbindung nach mindestens einem der vorangegangenen Ansprüche, wobei der unsubstituierte oder der substituierte aromatische Rest ein Phenyl-Rest, vorzugsweise ein einfach substituierter Phenyl-Rest, oder ein 4-Alkoxycarbonylphenyl-Rest ist.

10. Verbindung nach Anspruch 9, wobei der einfach substituierte Phenyl-Rest mit einem C₁-C₅-Alkyl-, einem Alkenyl-, einem Alkinyl-, einem Benzyl-, einem RO-, einem Halogen-, einem Formyl-, einem ROC-, einem RO₂C-, einem CN-, einem NO₂-, einem R-SO₂O-, einem RO-SO₂-, einem R-NH-SO₂-, einem R-SO₂-NH-, einem R-NH-CO-NH-, einem R-SO₂-NH-CO-NH-, einem R-NH-CO-NH-R- oder einem R-CO-NH-Rest substituiert ist, wobei R ein C₁-C₅-Alkyl-, ein Alkenyl-, ein Alkinyl-, ein Phenyl-, ein Tolyl- oder ein Benzyl-Rest ist.

11. Verbindung nach Anspruch 10, wobei der einfach substituierte Phenyl-Rest mit einem 4-C₁-C₅-Alkyl-, einem 4-RO- oder einem 4-(RO₂C)-Rest, wobei R ein C₁-C₅-Alkyl ist, substituiert ist.

12. Verbindung nach mindestens einem der vorangehenden Ansprüche, wobei Ar² ein substituierter Phenyl-Rest ist, der mit einem C₁-C₄-Alkyl-, einem Halogen-, einem CX₃-, einem Formyl-, einem ROC-, einem RO₂C-, einem CN-, einem NO₂- und/oder einem RO-Rest substituiert ist, wobei X ein Halogen-Rest und R ein C₁-C₅-Alkyl-, ein Phenyl- oder ein Tolyl-Rest ist.

13. Wärmeempfindliches Aufzeichnungsmaterial, umfassend ein Trägersubstrat, mindestens einen Farbbildner und mindestens einen phenolfreien Farbentwickler enthaltende wärmeempfindliche farbbildende Schicht, wobei der mindestens eine Farbentwickler die Verbindung der Formel (I) nach mindestens einem der Ansprüche 1 bis 12 ist.

14. Wärmeempfindliches Aufzeichnungsmaterial nach Anspruch 13, wobei der mindestens eine Farbbildner ein Farbstoff vom Triphenylmethan-Typ, vom Fluoran-Typ, vom Azaphthalid-Typ und/oder vom Fluoren-Typ, bevorzugt vom Fluoran-Typ, ist.

15. Wärmeempfindliches Aufzeichnungsmaterial nach mindestens einem der Ansprüche 13 und 14, wobei neben der Verbindung der Formel (I) ein oder mehrere weitere nicht-phenolische Farbentwickler vorliegen.

16. Wärmeempfindliches Aufzeichnungsmaterial nach mindestens einem der Ansprüche 13 bis 15, wobei die Verbindung der Formel (I) nach mindestens einem der Ansprüche 1 bis 12 in einer Menge von etwa 3 bis etwa 35 Gew.-%, bevorzugt von etwa 10 bis etwa 25 Gew.-%, bezogen auf den gesamten Feststoffgehalt der wärmeempfindlichen Schicht, vorliegt.

## Claims

1. A compound of formula (I),
Ar(NHSO₂Ar¹)ₗ(SO₂NHAr¹)ₘ(NHC(O)NHAr²)ₙ (I),
wherein
l and m independently of one another are 0, 1, 2, 3 and/or 4 and the sum of 1+m is equal to or greater than 1,
n is 2, 3, 4 or 5,
Ar is benzene group substituted (l+m+n) times,
Ar¹ is an unsubstituted or substituted aromatic group, and
Ar² is an unsubstituted or substituted phenyl group or a benzoyl group.

2. The compound according to claim 1, wherein 1 is 0 or 1.

3. The compound according to at least one of the preceding claims, wherein m is 0, 1 or 2.

4. The compound according to at least one of the preceding claims, wherein n is 2.

5. The compound according to at least one of the preceding claims, wherein 1 is 1, m is 0, and n is 2.

6. The compound according to at least one of the preceding claims, wherein 1 is 0, m is 1, and n is 2.

7. The compound according to at least one of the preceding claims, wherein 1 is 0, m is 2, and n is 2.

8. The compound according to at least one of the preceding claims, wherein Ar is a benzene group substituted 3 or 4 times, preferably a benzene group substituted 3 times.

9. The compound according to at least one of the preceding claims, wherein the unsubstituted or the substituted aromatic group is a phenyl group, preferably a monosubstituted phenyl group, or a 4-alkoxy carbonyl phenyl group.

10. The compound according to claim 9, wherein the monosubstituted phenyl group is substituted with a C₁-C₅ alkyl, an alkenyl, an alkynyl, a benzyl, an RO, a halogen, formyl, an ROC, an RO₂C, a CN, an NO₂, an R-SO₂O, an RO-SO₂, an R-NH-SO₂, an R-SO₂-NH, an R-NH-CO-NH, an R-SO₂-NH-CO-NH, an R-NH-CO-NH-R or an R-CO-NH group, wherein R is a C₁-C₅ alkyl, an alkenyl, an alkynyl, a phenyl, a tolyl or a benzyl group.

11. The compound according to claim 10, wherein the monosubstituted phenyl group is substituted with a 4-C₁-C₅ alkyl, a 4-RO or a 4-(RO₂C) group, wherein R is a C₁ to C₅ alkyl group.

12. The compound according to at least one of the preceding claims, wherein Ar² is a substituted phenyl group which is substituted with C₁-C₄ alkyl, a halogen, a CX₃, a formyl, an ROC, an RO₂C, a CN, an NO₂ or an RO group, wherein X is a halogen group and R is a C₁-C₅ alkyl group, a phenyl group or a tolyl group.

13. A heat-sensitive recording material, comprising a carrier substrate, at least one colour former, and at least one heat-sensitive colour-forming layer containing phenol-free colour developer, wherein the at least one colour developer is the compound of formula (I) according to at least one of claims 1 to 12.

14. The heat-sensitive recording material according to claim 13, wherein the at least one colour former is a dye of the triphenylmethane type, of the fluorane type, of the azaphthalide type and/or of the fluorene type.

15. The heat-sensitive recording material according to at least one of claims 13 and 14, wherein, besides the compound of formula (I), one or more further non-phenolic colour developer(s) is/are present.

16. The heat-sensitive recording material according to at least one of claims 13 to 15, wherein the compound of formula (I) according to at least one of claims 1 to 12 is present in an amount of from approximately 3 to approximately 35 % by weight, preferably of from approximately 10 to 25 % by weight, in relation to the total solid content of the heat-sensitive layer.

## Revendications

1. Composé de la formule (I),
Ar(NHSO₂Ar¹)ₗ(SO₂NHAr¹)ₘ(NHC(O)NHAr²)ₙ (I),
dans lequel
l et m sont indépendamment l'un de l'autre 0, 1, 2, 3 et/ou 4 et la somme l + m est égale ou supérieure à 1,
n est 2, 3, 4 ou 5,
Ar est un radical benzène substitué (l + m + n) fois,
Ar¹ est un radical aromatique non substitué ou substitué, et
Ar² est un radical phényle non substitué ou substitué ou un radical benzoyle.

2. Composé selon la revendication 1, dans lequel 1 est 0 ou 1.

3. Composé selon au moins l'une quelconque des revendications précédentes,
dans lequel m est 0, 1 ou 2.

4. Composé selon au moins l'une quelconque des revendications précédentes,
dans lequel n est 2.

5. Composé selon au moins l'une quelconque des revendications précédentes, dans lequel 1 est 1, m est 0 et n est 2.

6. Composé selon au moins l'une quelconque des revendications précédentes,
dans lequel l est 0, m est 1 et n est 2.

7. Composé selon au moins l'une quelconque des revendications précédentes,
dans lequel l est 0, m est 2 et n est 2.

8. Composé selon au moins l'une quelconque des revendications précédentes, dans lequel Ar est un radical benzène substitué 3 fois ou 4 fois, de préférence un radical benzène substitué 3 fois.

9. Composé selon au moins l'une quelconque des revendications précédentes, dans lequel le radical aromatique non substitué ou le radical aromatique substitué est un radical phényle, de préférence un radical phényle substitué une fois, ou un radical 4-alkoxycarbonylphényle.

10. Composé selon la revendication 9, dans lequel le radical phényle substitué une fois est substitué avec un radical alkyle en C₁-C₅, un radical alcényle, un radical alkinyle, un radical benzyle, un radical RO, un radical halogène, un radical formyle, un radical ROC, un radical RO₂C, un radical CN, un radical NO₂, un radical R-SO₂O, un radical RO-SO₂, un radical R-NH-SO₂, un racial R-SO₂-NH, un radical R-NH-CO-NH, un radical R-SO₂-NH-CO-NH, un radical R-NH-CO-NH-R ou un radical R-CO-NH, dans lequel R est un alkyle en C₁-C₅, un radical alcényle, un radical alkinyle, un radical phényle, un radical tolyle ou un radical benzyle.

11. Composé selon la revendication 10, dans lequel le radical phényle substitué une fois est substitué avec un radical 4-alkyle en C₁-C₅, un radical 4-RO ou un radical 4-(RO₂C), dans lequel R est un alkyle en C₁ - C₅.

12. Composé selon au moins l'une quelconque des revendications précédentes, dans lequel Ar² est un radical phényle substitué, qui est substitué avec un radical alkyle en C₁-C₄, un radical halogène, un radical CX₃, un radical formyle, un radical ROC, un radical RO₂C, un radical CN, un radical NO₂ et/ou un radical RO, dans lequel X est un radical halogène et R est un radical alkyle en C₁-C₅, un radical phényle ou un radical tolyle.

13. Matériau d'enregistrement thermosensible, comprenant un substrat de support, au moins un agent chromogène et au moins une couche chromogène thermosensible contenant un révélateur chromogène sans phénol, dans lequel l'au moins un révélateur chromogène est le composé de la formule (I) selon au moins l'une quelconque des revendications 1 à 12.

14. Matériau d'enregistrement thermosensible selon la revendication 13, dans lequel l'au moins un agent chromogène est un colorant du type triphénylméthane, du type fluorane, du type azaphthalide et/ou du type fluorène, de manière préférée du type fluorane.

15. Matériau d'enregistrement thermosensible selon au moins l'une quelconque des revendications 13 et 14, dans lequel outre le composé de la formule (I) un ou plusieurs autres révélateurs chromogènes non phénoliques sont présents.

16. Matériau d'enregistrement thermosensible selon au moins l'une quelconque des revendications 13 à 15, dans lequel le composé de la formule (I) est présent selon au moins l'une quelconque des revendications 1 à 12 en une quantité d'environ 3 à environ 35 % en poids, de manière préférée d'environ 10 à environ 25 % en poids, par rapport à la teneur totale en matière solide de la couche thermosensible.
